(19) Europäisches Patentamt
European Patent Office
Office européen des brevets

(11) **EP 2 400 859 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**01.07.2020   Patentblatt 2020/27**

(45) Hinweis auf die Patenterteilung:
**25.10.2017   Patentblatt 2017/43**

(21) Anmeldenummer: 10707826.3

(22) Anmeldetag: **26.02.2010**

(51) Int Cl.:
*A61Q 19/00* (2006.01)     *A23K 50/80* (2016.01)
*A23K 10/30* (2016.01)     *A23K 20/147* (2016.01)
*A23L 33/185* (2016.01)     *A61K 8/64* (2006.01)
*A23J 1/14* (2006.01)

(86) Internationale Anmeldenummer:
**PCT/EP2010/001222**

(87) Internationale Veröffentlichungsnummer:
**WO 2010/097238 (02.09.2010 Gazette 2010/35)**

(54) **VERFAHREN ZUR GEWINNUNG VON PROTEINPRÄPARATEN AUS SONNENBLUMENSAMEN**

PROCESS FOR THE PRODUCTION OF PROTEIN PREPARATIONS FROM SUNFLOWER SEEDS

PROCEDE DE PRODUCTION DE PREPARATIONS PROTEIQUES A BASE DE GRAINES DE TOURNESOL

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **27.02.2009   DE 102009010813**

(43) Veröffentlichungstag der Anmeldung:
**04.01.2012   Patentblatt 2012/01**

(60) Teilanmeldung:
**17197218.5 / 3 295 803**

(73) Patentinhaber: **Fraunhofer-Gesellschaft zur Förderung der angewandten Forschung e.V. 80686 München (DE)**

(72) Erfinder:
- **PICKARDT, Claudia 12203 Berlin (DE)**
- **EISNER, Peter 85354 Freising (DE)**
- **BADER, Stephanie 85356 Freising (DE)**
- **WILD, Florian 85354 Freising (DE)**
- **MÜLLER, Klaus 85356 Freising (DE)**

(74) Vertreter: **Gagel, Roland Patentanwalt Dr. Roland Gagel Landsberger Strasse 480a 81241 München (DE)**

(56) Entgegenhaltungen:
WO-A2-2004/000032     DD-A1- 269 086
DE-A1- 1 442 083     DE-A1- 2 545 043
DE-A1- 3 235 847     DE-A1- 3 707 541
US-A- 3 734 901     US-A- 4 219 469
US-A- 4 219 470

- BOURGES R H ET AL: "Obtencion de harina y de un concentrado proteinico a partir de semillas de heliantus annus y su incorporacion en galletas" ARCHIVOS LATINOAMERICANOS DE NUTRICION, Bd. 30, Nr. 4, 1980, Seiten 564-579, XP009135815
- SAEED M ET AL: "Sunflower protein concentrates and isolates low in polyphenols and phytate." JOURNAL OF FOOD SCIENCE, Bd. 53, Nr. 4, Juli 1988 (1988-07), Seiten 1127-1131, XP002590909 DOI: 10.1111/j.1365-2621.1988.tb13545.x
- GASSMANN B: "Preparation and application of vegetable proteins, especially proteins from sunflower seed, for human consumption. An approach." NAHRUNG, Bd. 27, Nr. 4, 1983, Seiten 351-369, XP002590910 DOI: 10.1002/food.19830270408
- TRANCHINO L ET AL: "Almost complete dehulling of high oil sunflower seed." JOURNAL OF THE AMERICAN OIL CHEMISTS' SOCIETY, Bd. 61, Nr. 7, Juli 1984 (1984-07), Seiten 1261-1265, XP002590911 DOI: 10.1007/BF02636267
- MILLER N ET AL: "A process for the dehulling of high oil-content sunflower seeds." FETTE SEIFEN ANSTRICHMITTEL, Bd. 88, Nr. 7, 1986, Seiten 268-271, XP002590912 DOI: 10.1002/LIPI.19860880708

- Lin M J Y: "Certain Functional Properties of Sunflower Meal Products" Journal of Food Science, [Online] Bd. 39, Nr. 2, 1974, - 1974 Seiten 368-370, XP002590913 ISSN: 0022-1147 Gefunden im Internet: URL:http://www3.interscience.wiley.com/cgi -bin/fulltext/119662970/PDFSTART?CRETRY=1& SRETRY=0> [gefunden am 2010-07-08]
- MIETH G ET AL: "Sunflower seeds and products of their processing. I. Production and value-determining constituents. (translated) TIOL- Sonnenblumensamen und deren Verarbeitungsprodukte. I. Aufkommen und wertbestimmende Bestandteile." DIE NAHRUNG, Bd. 28, Nr. 5, 1984, Seiten 533-577, XP002590914 DOI: 10.1002/FOOD.19840280509
- BOURGES R H et al.: "Obtencion de harina y de un concentrado proteinico a partir de semillas de heliantus annus y su incorporacion en galletas", ARCHIVOS LATINOAMERICANOS DE NUTRICION, vol. 30, no. 4, 1980, pages 564-579,
- MILLER N et al.: "A process for the dehulling of high oil-content sunflower seeds", FETTE SEIFEN ANSTRICHMITTEL, vol. 88, no. 7, 1986, pages 268-271,
- "Presskuchen/Expeller", Sanderlandshop, Retrieved from the Internet: URL:http://www.sanderlandshop.de/epages/15 511032.sf/de_DE/?ObjectPath=/Shops/1551103 2/Categories/Expeller
- MIETH G. et al.: "Sonnenblumensamen und deren Verarbeitungsprodukte 2. Mitt. Gewinnung proteinangereicherter Verarbeitungsprodukte", Die Nahrung, vol. 28, no. 9, 1984, pages 907-931,
- PAVLIK, ROBERT P et al.: "Modern Practices in Sunflower Oil Extraction", Oils & Fats International, vol. 5, 1991, pages 32-33,

**EP 2 400 859 B2**

## Beschreibung

### Technisches Anwendungsgebiet

Die vorliegende Erfindung betrifft ein Verfahren zur Gewinnung von Proteinpräparaten aus Sonnenblumensamen sowie mit dem Verfahren herstellbare Proteinpräparate mit verbesserten Anwendungseigenschaften.

### Stand der Technik

Proteinpräparate finden vielfach Anwendung in Lebensmitteln als ernährungsphysiologisch oder technofunktionell aktive Zutaten. Es gibt Proteinpräparate mit besonders hoher Proteinwertigkeit zur Anwendung als hochwertige Nahrungsmittelzusätze (Babynahrung, Spezialernährung, Sportlernahrung). Diese sind prinzipiell auch für die Formulierung von Futtermitteln von Interesse, in denen eine hohe Proteinverfügbarkeit gewährleistet werden muss. Andere Proteinpräparate haben eine gute Technofunktionalität und sind z.B. dafür geeignet, Schäume oder Emulsionen zu stabilisieren oder Gele auszubilden. Diese Proteinpräparate sind vorrangig als Lebensmittelzutaten geeignet und finden außerdem Anwendung für Spezial-Futtermittel oder technische Zwecke.

Grundsätzlich können Proteinpräparate tierischen und pflanzlichen Ursprungs unterschieden werden. Beispiele von Proteinpräparaten tierischen Ursprungs sind solche aus Hühnerei, Milch, Molke oder Kasein und Gelatinepräparate aus Schlachtabfällen. Nachteilig ist, dass solche Proteinpräparate einen charakteristischen Eigengeschmack und Eigengeruch aufweisen und daher auf bestimmte Anwendungen beschränkt sind. Oft sind sie teuer in der Herstellung und problematisch in Bezug auf Allergien und werden von bestimmten Verbrauchern aus ethischen Gründen abgelehnt.

Man unterscheidet bei Pflanzenproteinpräparaten aufgrund der Herstellung und des sich daraus ergebenden Proteingehalts zwischen Proteinkonzentraten und Proteinisolaten. Im Vergleich zu Pflanzenproteinkonzentraten mit einem Proteingehalt zwischen 60 % und 90 % haben Proteinisolate einen sehr hohen Proteingehalt, der mindestens 90 % beträgt. Zur Herstellung von Proteinisolaten werden die Proteine in Wasser gelöst und anschließend aus der wässrigen Lösung isoliert. Sie haben daher gegenüber den extrahierten Pflanzensamen ein verändertes Aminosäureprofil und veränderte Nährwert- und technofunktionelle Eigenschaften.

Als pflanzliche Proteinpräparate sind am Markt hauptsächlich Sojaproteinpräparate, nämlich Sojaproteinkonzentrate und -isolate, und Weizenkleberpräparate verfügbar. Daneben werden Proteinpräparate aus anderen Leguminosenproteinen, wie z.B. Erbsenproteinkonzentrate, angeboten.

Bekannt sind ebenfalls pflanzliche Proteinpräparate, Proteinkonzentrate und Proteinisolate, aus entölten Ölsamen, wie z.B. Rapssamen und Sonnenblumenkernen. Diese werden jedoch zurzeit fast ausschließlich zur Ölgewinnung genutzt. Die dabei anfallenden Press- und Extraktionsrückstände (Presskuchen und Schrote) werden bislang trotz des hohen ernährungsphysiologischen und technofunktionellen Potenzials im Gegensatz zu Soja nicht im Lebensmittelbereich verwertet. Ein Grund hierfür ist der Anteil störender Begleitstoffe, wie Polyphenole, die den Geschmack und die Farbe der Produkte beeinträchtigt.

Ölsamen und Leguminosen werden nach Stand der Technik mit Hexan entölt. Leguminosensamen werden dazu geschält, flockiert und in einer Extraktionsanlage mit Hexan extrahiert. Ölsamen werden alternativ flockiert und direkt entölt oder mechanisch teilentölt (Vorpressung) und anschließend durch Extraktion fertig entölt, wobei der Presskuchen vor der Extraktion aufgebrochen werden muss, um die Extraktion zu ermöglichen. Auch eine Fertigpressung bis auf Restölgehalte von ca. 5% ohne nachfolgende Extraktion wird durchgeführt, wobei der Restölgehalt in den Expellern (Presskuchen, Schilfern) die Lagerstabilität senkt.

Sonnenblumenkerne werden bisher überwiegend ungeschält oder nur zu maximal 2/3 geschält zur Entölung eingesetzt. Insbesondere zur Pressung, d.h. Fertigpressung oder Vorpressung als Teilentölung, wird ein hoher Schalenanteil als notwendig erachtet. Die Presskuchen und Schrote sind in diesen Fällen dunkel gefärbt und haben einen sehr hohen Rohfasergehalt. Sie eignen sich daher nicht zur Herstellung von hochwertigen Proteinmehlen und -konzentraten.

Es gibt unterschiedliche Ansätze, Proteine aus den Rückständen der Sonnenblumenölgewinnung zu isolieren. Die Entfernung von störenden Polyphenolen, hauptsächlich Chlorogensäure, die die Farbe von Sonnenblumen-Proteinisolaten beeinträchtigt, steht dabei im Vordergrund. Hierfür wurden bislang Extraktionen mit unterschiedlichen Lösungsmitteln, darunter auch Wasser und Alkohole zur Entfernung der Polyphenole aus entölten Sonnenblumenschroten vorgeschlagen. Die Gewinnung von Proteinisolaten aus Sonnenblumenkernen und -presskuchen oder -schroten ist besonders schwierig wegen der geringen Wasserlöslichkeit der Sonnenblumenproteine, die den Einsatz von Alkali oder Salzen erfordert. Dies zieht einen besonders hohen Wasserverbrauch zur Proteinaufbereitung (Waschen) nach sich, verbunden mit hohen Proteinverlusten, was die Herstellungskosten der Proteinisolate erhöht und damit ihr Applikationsspektrum einschränkt.

Zur Entfernung der farbaktiven phenolischen Substanzen aus entfetteten Sonnenblumenkernen mit dem Ziel der anschließenden Proteinextraktion und Gewinnung von Proteinisolaten aus dem so vorbehandelten Material wurden

3

unterschiedliche wässrige alkoholische Mischungen getestet, insbesondere Butanol in unterschiedlichen Anteilen mit salzsaurem Wasser, Ethanol mit einem Anteil von 95 % (v/v), Isopropanol (70 %, v/v) und Methanol (80 % v/v). Nachteilig bei der Extraktion mit diesen Lösemitteln ist die weitgehende Denaturierung der Proteine durch die Lösemittelbehandlung, so dass die Proteinlöslichkeit stark herabgesetzt wird. Dadurch sind die nachfolgende Extraktion von Proteinen bei der Herstellung von Proteinisolaten sowie deren Funktionalität stark eingeschränkt.

In der WO02/060273A1 wird ein Verfahren beschrieben, mit dem Proteinisolate mit einem Proteingehalt von mehr als 90% aus Sonnenblumensamen gewonnen werden. Die Proteine werden dafür wässrig extrahiert und durch eine Fällung mit Alkohol bei niedrigen Temperaturen gewonnen. Diese sind aufgrund des hohen Energieaufwandes für die Kühlung teuer und daher in ihrer Anwendung begrenzt. Im Artikel 'Obtencion de harina y de un concentrado proteinico a partir de semillas de Heliantus annus y su incorporacion en galletas' Hector Bourges R. et al., Archivos latinoamericanos de nutrición, Vol. XXX, No. 4, 1980, Seite 564-579, wird die Herstellung von Proteinkonzentraten aus Sonnenblumensamen beschrieben die unter anderem durch Schälen der Sonnenblumensamen, Wärmebehandlung, Pressen, Vermahlen des Presskuchens und Lösungsmittelextraktion mittels Hexan erhalten werden.

Die Herstellung von Proteinkonzentraten und -isolaten aus Sonnenblumenkernen mittels Lösungsmittelextraktion wird in 'Sunflower Protein Concentrates and Isolates Low in Polyphenols and Phytate', Mohammad Saeed, Munir Cheryan, Journal of Food Science, Vol. 53, No. 4, 1998, Seite 1127-1131, beschrieben.

Die Vorteile möglichst vollständigen Schälens von Sonnenblumensamen in der Herstellung von Proteinpräparaten wird in 'Preparation and application of vegetable proteins, especially proteins from sunflower seed, for human consumption. An approach', B. Gassmann, Die Nahrung, Vol. 27, Nr. 4, 1983, Seiten 351-369 sowie in 'Almost Complete Dehulling of High Oil Sunflower Seed', L. Tranchino et al., JAOCS, Vol. 61, No. 7, 1984, Seite 1261-1265, beschrieben. Proteinkonzentrate aus Sonnenblumenkernen werden durch trocken- oder nasstechnische Aufbereitung gewonnen, wobei das Protein im Rückstand verbleibt. Der hohe Anteil an unerwünschten Begleitstoffen schränkt ihre Verwendung im Lebensmittelbereich ein. Insgesamt sind die bekannten Pflanzenproteinkonzentrate, welche einen niedrigen Aufreinigungsgrad aufweisen, eingeschränkt in ihrer Funktionalität und/oder enthalten einen gewissen Anteil störender Komponenten, die den Nährwert, die Farbe, den Geruch und/oder Geschmack der sie enthaltenden Lebens- oder Nahrungsmittel sehr negativ beeinflussen können. Proteinkonzentrate aus Sonnenblumenkernen haben daher eine beschränkte Applikationsbreite und sind nur in niedrigen Konzentrationen einsetzbar.

Die Aufgabe der vorliegenden Erfindung besteht darin, ein kostengünstiges Verfahren zur Herstellung von Proteinpräparaten bereitzustellen, die sensorisch ansprechend und vielseitig einsetzbar sind.

## Darstellung der Erfindung

Diese Aufgabe wird mit dem Verfahren nach Anspruch 1 gelöst. Die weiteren Ansprüche geben bevorzugte Ausführungsformen des Verfahrens an. Bei dem vorgeschlagenen Verfahren zur Gewinnung der Proteinpräparate aus Sonnenblumensamen werden wenigstens folgende Schritte durchgeführt:

- Schälen der Sonnenblumensamen bis auf einen Restschalengehalt von ≤ 5 Gew.% oder Bereitstellen von geschälten Sonnenblumensamen mit einem Restschalengehalt von ≤ 5 Gew.% (jeweils bezogen auf die Gesamtmasse der Kernfraktion, wie sie unmittelbar nach dem Schälen erhalten wird);
- mechanische Teilentölung der geschälten Sonnenblumensamen durch Pressen, dabei Kontrolle der Temperatur auf unter 80°C, wobei zur Pressung eine Schneckenpresse oder ein Extruder verwendet wird und die mechanische Teilentölung bis auf einen Fett- oder Ölgehalt der geschälten Sonnenblumensamen im Bereich zwischen 10 und 35 Gew.% durchgeführt wird, bei dem durch das Pressen mit der Schneckenpress oder dem Extruder ein Presskuchen in der Form von Pellets oder Strängen erhalten wird, der eine Dicke im Bereich zwischen 0,2 und 4 cm aufweist; und
- Durchführung eines oder mehrerer Extraktionsschritte mit mindestens einem Lösungsmittel, wobei mindestens ein Extraktionsschritt am Presskuchen in der Form von Pellets oder Strängen durchgeführt wird, und durch die ein entfettetes proteinhaltiges Mehl als Proteinpräparat erhalten wird. Mindestens einer der Extraktionsschritte wird bei dem Verfahren so ausgeführt, dass eine weitere Entölung der teilentölten geschälten Sonnenblumensamen bewirkt wird.

Durch die Kombination des geringen Restschalengehaltes und der mechanischen Teilentölung bis auf den angegebenen Restölgehalt können Proteinkonzentrate erhalten werden, die sowohl optisch als auch funktionell für einen Einsatz im Lebens- oder Futtermittelbereich sehr vorteilhafte Eigenschaften aufweisen. Das Verfahren ermöglicht eine besonders schonende Behandlung der Proteine, indem bei der mechanischen und/oder weiteren Entölung eine zu hohe Temperatur vermieden wird, die zu unerwünschten Proteinveränderungen und Aromaveränderungen führen könnte.

Die mechanische Teilentölung der Sonnenblumenkerne auf die angegebenen Restölgehalte wird vorzugsweise so durchgeführt, dass ein mechanisch stabiler Presskuchen mit einer Dicke im Bereich zwischen 0,5 und 2 cm erhalten wird. Dadurch werden die anschließenden Verfahrensschritte vereinfacht, da aufgrund der Porosität und Dicke des Presskuchens auf eine mechanische Zerkleinerung vor der weiteren Extraktion verzichtet werden kann.

Das erfindungsgemäße Verfahren erlaubt auch eine schonende Herstellung des Präparates in der Art, dass eine Denaturierung der Proteine in definierter Weise zugelassen wird. Die Teilentölung und der eine oder die mehreren Extraktionsschritte werden dabei so durchgeführt, dass der Denaturierungsgrad der Proteine im entfetteten proteinhaltigen Mehl (bezogen auf das Eingangsprodukt des Verfahrens) maximal 40%, vorzugsweise zwischen 10% und 30%, beträgt. Dies ermöglicht es, qualitativ und sensorisch hochwertige Proteinepräparate mit einem breiten Applikationsspektrum zu gewinnen.

Vorzugsweise wird die Extraktion mit einem Lösungsmittel oder Lösungsmittelgemisch in mehreren Extraktionsschritten durchgeführt, die eine Kombination von mindestens einem lipophilen Extraktionsschritt mit einem lipophilen Lösungsmittel bzw. Lösungsmittelgemisch und mindestens einem hydrophilen Extraktionsschritt mit einem hydrophilen Lösungsmittel bzw. Lösungsmittelgemisch umfassen. Weiterhin wird vorzugsweise die Konzentration des Extraktionslösungsmittels im letzten Extraktionsschritt soweit erhöht, dass eine anschließende Trocknung besonders einfach und schonend gestaltet werden kann.

Das mit dem Verfahren herstellbare Proteinpräparat aus Sonnenblumenkernen weist einen Proteingehalt von mindestens 50% auf. Es ist für eine kostengünstige Herstellung zugänglich, da eine hohe Aufreinigung, wie sie bei den Proteinisolaten erforderlich ist, vermieden werden kann.

Überraschenderweise weist das Proteinpräparat trotz des höheren Anteils an proteinfremden Stoffen Eigenschaften auf, die ähnlich wie die bekannten Proteinisolate aus diesen Rohstoffen oder sogar vielfältiger als diese sind. Aufgrund der hellen Farbe sowie des ausgewogenen technofunktionellen Spektrums in Form der wasserbindenden, ölbindenden sowie emulgierenden Funktionalität ist das Proteinpräparat vielseitig einsetzbar, u. a. in Lebens- und Futtermitteln, um Wasser und/oder Öl zu binden und/oder eine Emulsion zu bilden. Das Proteinpräparat ist geeignet, andere Präparate zu ersetzen, welche für diese Funktionalitäten bis anhin verwendet wurden und welche tierischen oder pflanzlichen Ursprungs sind wie Hühnerei, Milch, Soja in Form von Sojaproteinisolaten, etc.

Bereits in Form des besonders kostengünstig herstellbaren Sonnenblumenkernmehls, d.h. des aus dem Verfahren direkt erhaltenen, entfetteten proteinhaltigen Mehls, weist das Proteinpräparat überraschenderweise Eigenschaften hinsichtlich Farbe und Funktionalitäten auf, die es erlauben, das Proteinmehl direkt in zahlreichen Lebensmittel- und Futtermittelanwendungen einzusetzen.

Der Anwendungsbereich des Proteinpräparats kann noch weiter erstreckt werden, wenn das Proteinpräparat frei von den pflanzen- oder saateigenen Aromen ist, insbesondere wenn es im Wesentlichen geruchsfrei und/oder im Wesentlichen geschmacksneutral ist. Dadurch wird u. a. vermieden, dass es bei der Einarbeitung des Proteinpräparats in Lebens- und Futtermitteln zu einer unerwünschten Geschmacks- und Aromaänderung kommt.

Auch durch das Erhalten einer schaumbildenden Funktionalität kann der Anwendungsbereich erweitert werden, so dass das Proteinpräparat z. B. als Ersatz für Hühnereiklar oder andere schaumbildende Zusätze verwendet werden kann, um schaumartige Lebensmittel herzustellen.

Vorzugsweise weist das Proteinpräparat einen niedrigen Fettgehalt auf, wodurch eine gute Lagerstabilität des Proteinpräparats gewährleistet wird.

Weiter vorzugsweise weist das Proteinpräparat einen niedrigen Gehalt an Phytinsäure, Oligosacchariden und/oder Phenolsäuren auf. Dadurch ist der Gehalt an Stoffen reduziert, die bei der Verdauung die Verwertung von Nährstoffen beeinträchtigen können.

**Kurze Beschreibung der Zeichnungen**

Im Folgenden werden das vorgeschlagene Herstellungsverfahren sowie das damit herstellbare Proteinpräparat in Verbindung mit den Zeichnungen nochmals näher erläutert. Hierbei zeigen:

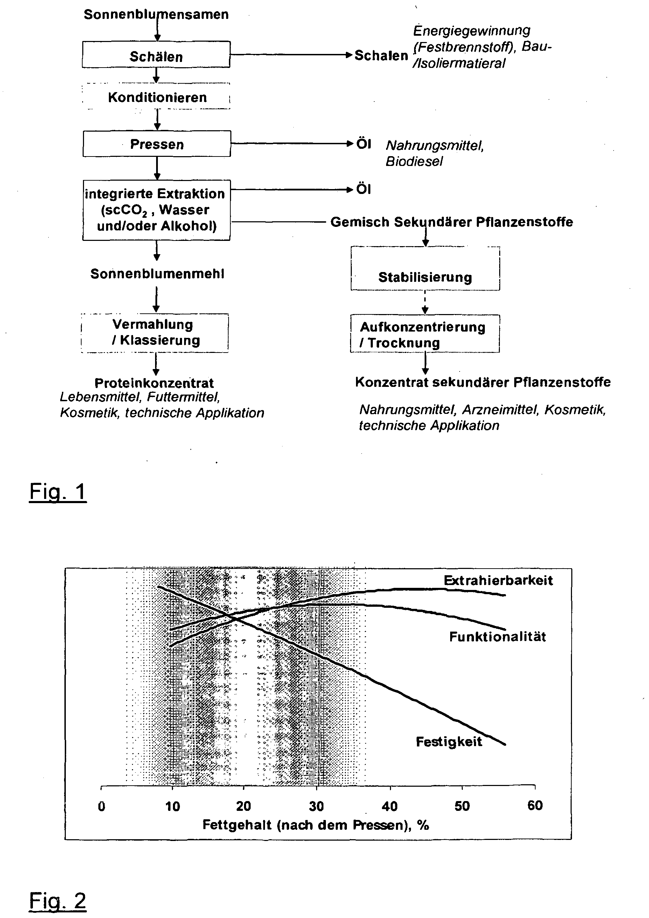

Fig. 1 schematisch ein Beispiel für den Verfahrens-ablauf des vorgeschlagenen Verfahrens mit einer Fraktionierung der phenolsäurehaltigen Sonnenblumenkerne in Öl, Polyphenole und Proteinkonzentrat sowie einer optionalen weiteren Fraktionierung in Proteinisolate; die Verwendungsmöglichkeiten der einzelnen Fraktionen sind in kursiver Schrift angegeben;

Fig. 2 eine Darstellung zur Ermittlung des optimalen Pressgrades hinsichtlich der mechanischen Stabilität und Erhalt der Extrahierbarkeit der Presskuchen und funktionellen Eigenschaften der daraus hergestellten Präparate; und

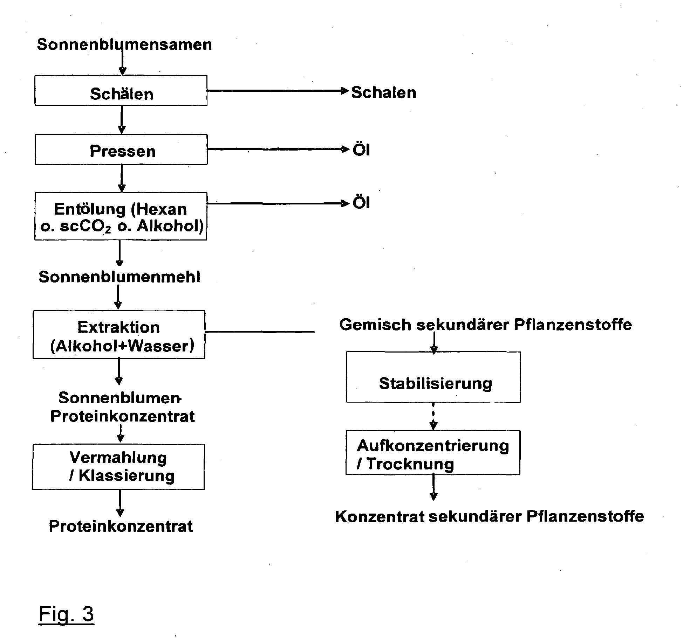

Fig. 3 schematisch ein weiteres Beispiel für den Verfahrensablauf des vorgeschlagenen Verfahrens mit den Schritten Schälen, Pressen und Hexanextraktion (alternativ: Entölung mit $scCO_2$ oder mit Alkohol), anschließender Alkohol-Wasser-Extraktion, Alkohol-Wasser-Verdrängungstrocknung und anschließender Feinvermahlung und/oder Sieben.

**Wege zur Ausführung der Erfindung**

Das erfindungsgemäße Verfahren kann beispielsweise in folgender Weise durchgeführt werden. Als Ausgangsprodukt werden bevorzugt Speisetype-Sonnenblumenkern-Sorten gewählt oder solche, die eine helle Schale haben. Es können aber auch normale und High oleic Öltype-Sonnenblumenkerne verwendet werden.

Der vorbereitete Rohstoff wird in einer Extraktionsvorrichtung nacheinander mit unterschiedlichen Lösungsmitteln unter solchen Bedingungen extrahiert, dass keine oder nur sehr wenig Proteine gelöst werden. Dadurch werden Proteinverluste und -veränderungen minimiert. Besonders vorteilhaft ist die Durchführung einer Lösemittelextraktion mit einem Alkohol, beispielsweise Ethanol, Propanol, Isopropanol.

Selbstverständlich können auch mehrere Extraktionsvorrichtungen für die unterschiedlichen Lösungsmittel eingesetzt werden. Das Gleiche gilt auch für die Trocknungseinrichtungen.

Das gesamte Verfahren umfasst die drei Schritte Auswahl und Aufbereitung der Rohstoffe, mechanische Teilentölung sowie Extraktion, und ist schematisch in Figur 1 dargestellt. Ein weiteres Ausführungsbeispiel ist der Figur 3 zu entnehmen.

1. Auswahl und Aufbereitung der Rohstoffe:

Weitgehende Abtrennung der Schalen durch eine geeignete Schältechnologie, so dass der Restschalengehalt in Bezug auf die erhaltene geschälte Kernfraktion bei ≤ 5 Gew%, bevorzugt bei ≤ 1 Gew% liegt. Besonders vorteilhaft werden hierzu gezielt leicht schälbare Rohstofftypen und -sorten ausgewählt, insbesondere Speisekerne anstatt Öltypekerne. Die Angaben zum Restschalengehalt beziehen sich in der vorliegenden Patentanmeldung auf die Gesamtmasse der Kernfraktion, wie sie unmittelbar nach dem Schälen erhalten wird.

Eine gezielte Konditionierung und/oder Trocknung stellt sicher, dass während der Entölung enzymatische Prozesse verhindert oder kontrolliert werden. Diese kann vor oder nach dem Schälen erforderlich sein.

2. mechanische Teilentölung

Pressen der schalenarmen Sonnenblumenkerne durch Schneckenpressen, dabei Kontrolle der Temperatur, ggf. Kühlung, auf unter 80°C, besser unter 60 °C, noch besser unter 50°C. Damit werden Maillardreaktionen und sonstige Proteinveränderungen vermindert, außerdem Reaktionen von anderen Begleitstoffen mit Proteinen, z.B. Polyphenole.

Das Pressen erfolgt bis auf einen Restölgehalt von 10 - 35 Gew.%, vorzugsweise 12 - 25% Gew.%, besonders bevorzugt zwischen 17 und 25 Gew.%. Die Pressung wird mit einer Pressgeometrie bzw. Düse durchgeführt, die die Ausformung von stabilen Presskuchen in der Form von Pellets oder Strängen ermöglicht, die dennoch nicht zu hart zusammengepresst sind und eine gewisse Porosität aufweisen.

Bei einer geeigneten Wahl der Presskonfiguration (insb. der Düsengeometrie) halten die Pellets bei den oben angegebenen Restölgehalten überraschenderweise sehr gut zusammen und ermöglichen trotz des geringen Schalenanteils ohne anschließende Zerkleinerung eine weitere Entölung mit einem Lösungsmittel. Die Erfinder haben hierbei herausgefunden, dass durch die obige mechanische Teilentölung in Schneckenpressen ein guter Saat-Aufschluss und eine vorteilhafte Produktform für die Extraktion erreicht werden, so dass auf eine weitere Zerkleinerung oder Vorbereitung für die anschließenden weiteren Extraktionsschritte verzichtet werden kann. Dabei wurde erkannt, dass der erzielte Restölgehalt mit den mechanischen Eigenschaften in der Art zusammenhängt, dass es einen optimalen Grad der Entölung gibt, bei dem die Presskucheneigenschaften ideal sind, wie dies aus Figur 2 ersichtlich ist. Der optimale Grad der Entölung liegt in diesem Beispiel bei einem Restölgehalt von etwa 17-20 Gew.%.

Besonders vorteilhaft für die Effizienz der weiteren Extraktionsschritte wird die mechanische Teilentölung bis auf einen Fett- oder Ölgehalt der geschälten Sonnenblumensamen durchgeführt, bei dem durch das Pressen ein stabiler Presskuchen erhalten wird, der eine Dicke im Bereich zwischen 0,5 und 2 cm aufweist.

Die Pressung wird mit einer Pressgeometrie bzw. Düse durchgeführt, die die Ausformung von stabilen Presskuchen in der Form von Pellets oder Strängen ermöglicht. Besonders vorteilhaft wird eine Schneckenpresse mit einer Rundloch-Matrize oder einer Düse oder ein Extruder mit Runddüse zur Pressung verwendet, so dass die erhaltenen Presslinge als Stränge mit rundem Querschnitt von 5 - 20 mm Durchmesser erhalten werden. Durch die Wahl eines geeigneten Pressgrads, bei dem der Restfettgehalt im Bereich zwischen 12 und 25 % liegt, werden Presslinge mit einer für die Extraktion noch ausreichenden Porosität und guter Festigkeit erhalten. Dabei werden Pressstränge mit einer Bruchfestigkeit zwischen 2 und 10 $N/mm^2$, idealerweise zwischen 4 und 8 $N/mm^2$ bei einem Schüttgewicht zwischen 300 und 500 $kg/m^3$ erhalten

3. Extraktion der vorbereiteten Samen bzw. der Presskuchen-Pellets:

Vorzugsweise erfolgt die weitere Extraktion durch Kombination von mindestens zwei Extraktionslösungsmitteln unterschiedlicher Polarität in der Art, dass enthaltene hydrophilere Begleitstoffe vor, mit oder nach dem Öl extrahiert werden. Als Extraktionslösungsmittel werden nachfolgend alle reinen Fluide und Lösungen (z.B. organische Lösungsmittel oder Wasser und wässrige Lösungen oder überkritische Gase) und Fluidgemische bezeichnet, die zur Extraktion verwendet werden. Dabei werden mindestens zwei Polariätswechsel durch Aufeinanderfolge der Extraktionslösungsmittel eingestellt. Diese können schlagartig oder kontinuierlich eingestellt werden, indem das vorher vorhandene Extraktionslösungsmittel mit dem nachfolgenden vermischt oder durch dieses verdrängt wird. Als solche kommen alle lebensmittelrechtlich zugelassenen Lösungsmittel und ihre Gemische in Frage, insbesondere Wasser, Säuren, Alkohole, Ester, Ketone, z.B. Aceton, Ether, Alkane wie n-Hexan und iso-Hexan, deren Polarität bzw. Wasserlöslichkeit in der genannten Reihenfolge grundsätzlich abnimmt (von hydrophil zu lipophil), sowie überkritische Flüssigkeiten und Gase, z.B. $scCO_2$ (überkritisches $CO_2$), das am kritischen Punkt eher lipophil ist und dessen Polarität durch weitere Erhöhung des Drucks in Richtung hydrophil sowie durch Erhöhung der Temperatur weiter verändert werden kann.

So können beispielsweise folgende Schritte durchgeführt werden, wobei die Reihenfolge der hydrophilen und lipophilen Schritte vorzugsweise so gewählt wird, dass die Gesamtextraktion eine maximale Ausbeute ergibt (d.h. mindestens 90% der mit dem reinen Lösungsmittel erzielbaren Ausbeute):

- Extraktion von mäßig hydrophilen Begleitstoffen, insbesondere Phenolsäuren und Aromastoffen, durch Alkohol, vorzugsweise Isopropanol, Ethanol oder Methanol, in einer Konzentration, bei der die Proteine nicht oder nur wenig gelöst werden. Dazu wird eine Alkoholkonzentration von größer 60%, vorzugsweise zwischen 60 und 80%, eingestellt (v/v-Konzentration des Alkohols im Extraktionslösungsmittel). Weiterhin kann auch $scCO_2$ als Lösungsmittel eingesetzt werden, vorzugsweise bei einer Temperatur zwischen 40 und 80°C und bei einem Druck über $300 * 10^5$ Pa, vorzugsweise im Bereich von $350 - 800 * 10^5$ Pa, wobei es mit zunehmendem Druck hydrophilere Eigenschaften aufweist.

- Extraktion lipophiler Bestandteile mit einem lipophilen Lösungsmittel bis zur vollständigen Entölung auf einen Restölgehalt von höchstens 5% (Büchi-Methode nach Caviezel). Als lipophiles Lösungsmittel kann beispielsweise Hexan, reiner Alkohol ($\geq$ 95%) oder $scCO_2$ bei einer Temperatur im Bereich von 31 - 60°C und einem Druck im Bereich von $74 - 350 * 10^5$ Pa eingesetzt werden. Damit werden insbesondere Öl, Phospholipide und andere lipophile Bestandteile wie beispielsweise Carotinoide extrahiert.

- ggf. Wiederholung der ersten Extraktion nach der zweiten Extraktion.

Die lipophile Extraktion kann bei Bedarf auch vor der hydrophilen Extraktion durchgeführt werden.

Vorteilhafterweise wird die Polarität der Extraktionslösungsmittel durch vorhandenes Restwasser nach der Vorbehandlung, insbesondere nach der mechanischen Vor-Entölung bzw. während der Ölextraktion verändert, so dass während des Extraktionsprozesses mit einem einzigen zugesetzten Lösungsmittel unterschiedliche Polaritäten des tatsächlichen Extraktionsgemischs entstehen.

Bei Nutzung von überkritischen Gasen, insbesondere überkritischem Kohlenstoffdioxid ($scCO_2$) kann die Polarität allein durch Änderung von Druck und Temperatur verändert werden, so dass der Zusatz eines weiteren Lösungsmittels für einen Polaritätswechsel nicht erforderlich ist. Durch sukzessive Verdrängung des rohstoffgebundenen Wassers kann die Polarität während der Extraktion nahezu kontinuierlich verändert werden.

Besonders vorteilhaft wird die hydrophilere Polarität zuerst eingestellt, so dass das im Rohstoff gebundene Restwasser zur Modifikation der Polarität in der Art genutzt werden kann, dass hydrophile Substanzen ohne weiteren Wasserzusatz oder mit sehr wenig zugesetztem Wasser extrahiert werden können. Überraschenderweise bewirkt dies beim Übergang zur lipophilen Extraktionsphase gleichzeitig eine Verminderung des Restwassergehalts, so dass die lipophile Extraktion begünstigt wird. Durch die Entfernung des Wassers vor bzw. während der ersten Extraktion kann eine sonst übliche Trocknung vor der Entölung entfallen. Normalerweise wäre nach dem Pressen eine Konditionierung erforderlich, weil bei angestiegenem relativen Wassergehalt im Presskuchen wegen verminderten Ölgehalts und resultierender geringerer Gesamtmasse die Entölung mit lipophilem Lösungsmittel erschwert wäre.

Besonders vorteilhaft wird das erste Lösungsmittel oder werden Reste des ersten Lösungsmittels, z.B. des Alkohols oder Alkohol-Wassergemischs, mit dem anschließenden zweiten Lösungsmittel ausgetrieben.

Ggf. kann es nötig sein, zuvor das Wasser mit Alkohol vollständig zu verdrängen, um nachteilige Einflüsse von Wasser in der nachfolgenden Extraktion zu verhindern. Die Alkoholkonzentration wird in der ersten Extraktion soweit erhöht, dass der Alkohol anschließend durch das lipophilere Lösungsmittel gelöst werden kann. Eine selektive Abtrennung bei unterschiedlichen Druckstufen beim Einsatz von $scCO_2$ ermöglicht die weitgehende Abtrennung der Alkoholphase. Durch das im Rohstoff enthaltene Wasser oder zugesetztes Wasser oder andere Co-Solvents können die Lösungseigenschaften weiter modifiziert werden, so dass die Gewinnung mäßig polarer Wertstoffe möglich ist. Durch Kombination von hohem Druck ($>500*10^5$ Pa) und einer Temperatur zwischen 40 und 60°C werden bessere Extraktionsraten der Phenolsäuren und der Ölbegleitstoffe erzielt. Die Einbringung des zweiten Lösungsmittels (außer Wasser) in die überkritische Phase kann die Extraktion der Phenolsäuren und weiterer Begleitstoffe, z.B. Pigmente und Aromastoffe auch verbessern. Besonders vorteilhaft werden die Extraktionsbedingungen mit $scCO_2$ so eingestellt, dass hintereinander sowohl Wasser- als auch Alkohol-Reste aus dem Raffinat ausgetrieben werden können und ein Desolventieren mit hohen Temperaturen dadurch überflüssig wird. Dadurch kann auf eine anschließende Trocknung des Raffinats auch bei Verwendung von Wasser als Schleppmittel/ Modifier verzichtet werden.

Bei der Verwendung von wässrigem Alkohol wird die Extraktion mittels des Extraktionslösungsmittels in mehreren Extraktionsschritten durchgeführt, wobei bei mindestens dem letzten Übergang von dem einem zum nächsten Extraktionsschritt der Alkoholgehalt im Extraktionslösungsmittel maximal erhöht wird, d.h. bis zur Konzentration des wässrigen Azeotrophs, z.B. 96 % (v/v) bei Ethanol, so dass die Alkoholkonzentration im Extraktionsgemisch auf über 90 % (v/v) ansteigt. Dies erlaubt es, die anschließende Trocknung besonders schonend zu gestalten, da der Anteil des zu entfernenden Restwassers, das weniger schnell und bei einer höheren Temperatur als der Alkohol verdampft, reduziert ist.

Selbstverständlich kann die weitere Extraktion (nach der mechanischen Teilentölung) auch mit nur einem Lösungsmittel, insbesondere Hexan, durchgeführt werden, um das entfettete proteinhaltige Mehl zu erhalten.

Die Extraktion wird unter solchen Bedingungen durchgeführt, dass Proteine nicht oder nur geringfügig in Lösung gehen und die Proteine nicht oder nur minimal geschädigt werden und keine oder nur wenige unerwünschte chemische Reaktionen auftreten wie Maillardreaktion oder Michael-Addition von Phenolsäuren (z.B. messbar als max. 20% weniger freie Phenolsäuren und/oder verfügbares Lysin und/oder reduzierbare Zucker bzw. max. 10% mehr Lysinoalanin oder Maillardprodukte). Weiterhin treten bei den eingestellten Temperaturen keine thermisch bedingten Aromaveränderungen des extrahierten Materials auf. Hierzu wird insbesondere die Temperatur unter 80°C, besser bei $\leq$ 60°C gehalten, idealerweise unter 40°C. Wenn eine Hexanentölung durchgeführt wird, kann die vollständige Desolventierung durch Anlegen eines Vakuums (100 - 800 hPa, vorzugsweise 200 - 500 hPa, besonders bevorzugt 200 hPa) verbessert werden, wodurch die Desolventierung bei Temperaturen bis maximal 60°C ermöglicht wird. Bei anderen Lösungsmitteln ist die Anwendung von Vakuum ggf. ebenfalls vorteilhaft, um die Desolventierung bei niedrigeren Temperaturen zu ermöglichen.

Dabei zeigt sich, dass bei Sonnenblumenproteinen eine definierte Denaturierung von 5% - 40%, besonders günstig zwischen 10% und 30% (z.B. messbar als Abweichung um maximal 30%, besser 20%, noch besser 10% hinsichtlich funktioneller Eigenschaften wie Proteinlöslichkeit, max. 30% größere Proteindenaturierung, messbar mit thermoanalytischen Methoden wie DSC), - bezogen auf Proteine des Eingangsprodukts des vorgeschlagenen Verfahrens - besonders vorteilhaft ist, um ein breites Applikationsspektrum zu erhalten.

Die Reihenfolge der Extraktion kann auch umgekehrt erfolgen, wenn z.B. die Extraktstoffe für spezifische Anwendungen nutzbar gemacht werden sollen. Die vollständige Entölung im ersten Schritt kann im Hinblick auf die Gewinnung der Begleitstoffe als funktionelle Lebensmittelzutaten oder für kosmetische oder technische Anwendungen vorteilhaft sein. Besonders vorteilhaft ist die Kombination von Entölung mit überkritischem $CO_2$, anschließender (wässrig-)alkoholischer Lösungsmittelextraktion und abschließender $scCO_2$-Behandlung zur gleichzeitigen Desolventierung und Trocknung zu stabilen Endprodukten. Die Kombination wird vorzugsweise so gestaltet, dass alle Extraktionen hintereinander in einem Behälter durchgeführt werden und nur die Lösungsmittel, Temperaturen und Drücke verändert werden.

Überraschenderweise können dabei gleichzeitig die wertvollen Fraktionen Proteine und Phenole gewonnen und beide für unterschiedliche Lebensmittelanwendungen genutzt werden. Die im Alkohol enthaltenen Begleitstoffe können direkt für hochwertige Anwendungen genutzt werden oder weiter verarbeitet werden. Besonders vorteilhaft ist auch die Verwendung von $scCO_2$ vor oder bei der Extraktion von Polyphenolen, weil durch die Verdrängung von Sauerstoff eine Oxidation verhindert wird.

Überraschenderweise zeigt sich auch, dass beim Einsatz von Alkohol die Abreicherung der Phospholipide gegenüber einer reinen Hexanextraktion verbessert wurde, wodurch die sensorische Qualität des entölten Mehls weiter verbessert wird.

Weiterhin zeigt sich, dass die Proteinfraktion frei von thermisch bedingten Aromastoffen gewonnen werden kann und die Anwendung in Lebensmitteln durch sensorisch neutrale Proteinpräparate verbessert wird. Gleichzeitig bleiben die funktionellen Eigenschaften der Proteine erhalten.

Durch das oben beschriebene Herstellungsverfahren kann ein Sonnenblumenproteinpräparat gewonnen werden, das sich z. B. gegenüber Proteinisolaten, die durch wässrige Fraktionierung und aufwändige Isolierungsverfahren gewonnen werden, durch ein ausgewogenes Nährwertprofil und technofunktionelles Spektrum auszeichnen. Das Proteinpräparat ist auch ohne weitere Aufbereitung, um z. B. den hohen Proteingehalt eines Proteinisolates zu erhalten, u. a. als Lebens- oder Futtermittelzutat geeignet. Überraschenderweise zeigt das Proteinpräparat, obwohl es kein Proteinisolat ist, technofunktionelle Eigenschaften von Proteinisolaten auf. Es hat eine neutrale, helle Farbe und ist weitgehend frei von sensorisch störenden und antinutritiven Begleitstoffen. Insbesondere weist das Sonnenblumenproteinkonzentrat nahezu keinen Eigengeruch und Eigengeschmack auf.

Besonders überraschend ist, dass bereits das entölte Sonnenblumenproteinmehl (SBPM) eine überaus ansprechende Farbe und sehr ausgeprägte Funktionalität hat und für zahlreiche Lebensmittel- und Futtermittelanwendungen geeignet ist.

Nachfolgend wird zur quantitativen Charakterisierung der hergestellten Proteinpräparate auf folgende Bestimmungsverfahren zurückgegriffen:

- Proteingehalt:

Der Proteingehalt ist definiert als der Gehalt, der sich aus der Bestimmung des Stickstoff und dessen Multiplikation mit dem Faktor 6,25 errechnet. Der Proteingehalt ist z. B. in Prozent bezogen auf die Trockenmasse (TS) angebbar.

- Farbe:

Die wahrnehmbare Farbe ist mittels CIE-L*a*b*-Farbmessung definiert (vgl. DIN 6417). Dabei gibt die L*-Achse die Helligkeit an, wobei Schwarz den Wert 0 und Weiss den Wert 100 hat, die a*-Achse beschreibt den Grün- oder Rotanteil und die b*-Achse den Blau- oder Gelbanteil.

- Proteinlöslichkeit:

Die Proteinlöslichkeit ist mittels Bestimmungsverfahren nach Morr et al. 1985 bestimmt (siehe den Zeitschriftenartikel: Morr C.V., German, B., Kinsella, J.E., Regenstein, J.M., Van Buren, J.P., Kilara, A., Lewis, B.A., Mangino, M.E, "A Collaborative Study to Develop a Standardized Food Protein Solubility Procedure. Journal of Food Science", Band 50 (1985) Seiten 1715-1718).Dabei wird das Proteinpräparat zu einem Masse-Volumenanteil von 1:25 bis 1:50 (w/v) (d. h. 1-2 g des Proteinpräparats auf 50 ml Lösung) in einer 0,1 M NaCl-Lösung bei Raumtemperatur suspendiert und unter Verwendung von 0,1 M HCl- oder NaOH-Lösung ca. 60 min bei einem pH-Wert von pH 7 gehalten und mit ca. 200 U/min gerührt und das unlösliche Sediment danach für 15 min bei 20tausendfacher Erdbeschleunigung (20'000g) abzentrifugiert. Die Proteinlöslichkeit ist z. B. in Prozent angebbar, wobei eine Proteinlöslichkeit von x % bedeutet, dass x % des im Präparat vorhandenen Proteins im geklärten Überstand wiedergefunden werden, wenn die genannte Methode angewendet wird.

- Wasserbindung:

Das Wasserbindevermögen ist mittels Bestimmungsverfahren (nachfolgend AACC-Bestimmungsverfahren genannt) definiert, wie es angegeben ist in: American Association of Cereal Chemists, "Approved methods of the AACC". 10th ed., AACC. St. Paul, MN, 2000b; Method 56-20. "Hydration capacity of pregelatinized cereal products". Das Wasserbindevermögen ist z. B. in ml/g angebbar, d. h. Milliliter gebundenes Wasser pro Gramm Präparat, und wird gemäss dem AACC-Bestimmungsverfahren bestimmt über das Gewicht des mit Wasser gesättigten Sediments abzüglich der Einwaage des trockenen Präparats nach Mischung von ca. 2 g Proteinpräparat mit ca. 40 ml Wasser für 10 Minuten und Zentrifugation bei 1'000g für 15 Minuten bei 20°C.

- Ölbindung:

Das Ölbindevermögen ist mittels Bestimmungsverfahren (nachfolgend Fettbinde-Bestimmungsverfahren genannt) definiert, wie es angegeben ist in: Ludwig I., Ludwig, E., Pingel B., "Eine Mikromethode zur Bestimmung der Fettbindkapazität". Nahrung/Food, 1989, 33(1), 99.
Das Ölbindevermögen ist z. B. in ml/g angebbar, d. h. Milliliter gebundenes Öl pro Gramm Präparat, und wird gemäß o.g. Bestimmungsverfahren gemessen als Volumen des ölbindenden Sediments nach Mischung von 1,5 g Proteinpräparat mit 15 ml Maiskeimöl für 1 Minute und Zentrifugation bei 700g für 15 Minuten bei 20°C.

- Emulgierkapazität:

Die Emulgierkapazität ist mittels Bestimmungsverfahren (nachfolgend Konduktivitätsmessungs-Methode genannt) bestimmt, bei welchem einer 1 %igen Suspension des Proteinpräparats von 100 ml, pH 7, Maiskeimöl zugegeben wird bis zur Phaseninversion der Öl-in-Wasser-Emulsion. Die Emulgierkapazität ist definiert als das maximale Ölaufnahmevermögen dieser Suspension, bestimmt über die spontane Abnahme der Leitfähigkeit bei der Phaseninversion (vgl. den Zeitschriftenartikel von Wäsche, A., Müller, K., Knauf, U., "New processing of lupin protein isolates and functional properties". Nahrung/Food, 2001, 45, 393-395) und ist z. B. angebbar in ml Öl/g, d. h. Milliliter emulgiertes Öl pro Gramm Proteinpräparat

- Schaumaktivität:

  Die Schaumaktivität ist angegeben in Prozent, gemessen als Volumenzunahme einer 5 %igen Lösung, pH 7, bei Aufschlag während 8 min auf Stufe 3 (591 U/min) in einer Hobart 50N Standard-Küchenmaschine (Stahlkessel mit 5 Liter Inhalt) mit Rührbesen (Drahtbesen).

- Schaumdichte:

  Die Schaumdichte ist angegeben in g/l, d. h. Masse des Schaums pro Volumeneinheit, und wird gemessen nach Aufschlag einer 5 %igen Lösung, pH 7, von 8 min auf Stufe 3 (591 U/min) in einer in einer Hobart 50N Standard-Küchenmaschine (Stahlkessel mit 5 Liter Inhalt) mit Rührbesen (Drahtbesen).

- Schaumstabilität:

  Die Schaumstabilität ist angegeben in Prozent, gemessen als Volumenabnahme von 100 ml Schaum innerhalb einer Stunde nach Aufschlag einer 5 %igen Lösung, pH 7, 8 min auf Stufe 3 (591 U/min) in einer Hobart 50N Standard-Küchenmaschine (Stahlkessel mit 5 Liter Inhalt) mit Rührbesen (Drahtbesen).

- Fettgehalt:

  Der Fettgehalt ist bestimmt nach Probenaufschluss und Verseifung der Fettsäuren z. B. nach der Caviezel-Methode (beschrieben bei DGF. "Method of Caviezel", DGF K-I 2c (00). In Deutsche Gesellschaft für Fettwissenschaft e. V., Münster. DGF-Einheitsmethoden, 2nd edition. Stuttgart: WVG, 2004).

- Für Vergleichszwecke wurden folgende kommerzielle hergestellte Produkte verwendet:

  - Erbsenproteinisolat Pisane® (hergestellt von Cosucra),
  - Sojaproteinisolat SUPRO® EX33 (hergestellt von DuPont).
  - Natriumkaseinat (sprühgetrocknet), FN5S von Rovita.

Mit dem erfindungsgemäßen Herstellungsverfahren sind Proteinpräparate aus Sonnenblumenkernen mit folgenden Eigenschaften herstellbar:

Aussehen:

- In schüttfähiger Form, z. B. als Flocken, Granulat, Pulver oder in Form anderer Partikel.
- Die Farbe ist weiß bis cremefarben, hellgrau oder hellgelb, gegebenenfalls mit einem Anteil heller oder dunkler gefärbter Partikel von maximal 5 % w/w, vorzugsweise unter 2 % w/w. Die Helligkeit L* gemäß CIE-L*a*b*-Farbmessung ergibt einen Wert von mindestens 70, L* >= 70. Folgendes sind typische Werte für L*, a* und b*:

$$L^* >= 80, \quad -5 < a^* < +5, \quad -5 < b^* < +20;$$

vorzugsweise

$$L^* >= 85, \quad -3 < a^* < +3, \quad -2 < b^* < +15;$$

besonders bevorzugt L* >= 90, -1 < a* < +1, 0 < b* < +10.

Zusammensetzung:

- Der Proteingehalt beträgt weniger als 90 % in der Trockenmasse (TS), vorzugsweise weniger als 80 % bezogen auf TS. Typischerweise liegt der Proteingehalt zwischen 50 und 70 % bezogen auf TS.

- Gesamtballaststoffgehalt zwischen 10 und 40 % bezogen auf TS, bevorzugt zwischen 10 und 30 % bezogen auf TS.
- Fettgehalt, ermittelt z. B. durch gravimetrische Bestimmung nach Soxhletextraktion, unter 3 % bezogen auf TS, bevorzugt unter 1 %.
- Gesamtzuckergehalt unter 15 % bezogen auf TS, bevorzugt unter 5 %, besonders bevorzugt unter 2 %.
- Gehalt an unerwünschten, insbesondere antinutritiven Stoffen:

  • Phytinsäuregehalt unter 5 % bezogen auf TS, bevorzugt unter 2 %, besonders bevorzugt unter 1 %.
  • Raffinosegehalt unter 5% bezogen auf TS, bevorzugt unter 2,5%, besonders bevorzugt unter 0,5%.
  • Phenolsäuregehalt (bestimmt als Chlorogensäure) unter 5 % bezogen auf TS, bevorzugt unter 2 %, besonders bevorzugt unter 0,5 %.

- Ligningehalt unter 6 % bezogen auf TS, bevorzugt unter 4 %, besonders bevorzugt unter 3 %.
- Allgemein ist der Protein- sowie Ligningehalt beim Sonnenblumenproteinmehl (SBPM) tiefer als beim daraus hergestellten Sonnenblumenproteinkonzentrat (SBPK), während der Gehalt an Fett, Zuckern und Phenolsäuren beim SBPM höher als beim SBPK ist.

Technofunktionelle Eigenschaften:

- Proteinlöslichkeit:

  Die Proteinlöslichkeit, bestimmt nach dem PNG-Bestimmungsverfahren, ist größer als 30 %, bevorzugt größer als 40 %. Typischerweise liegt die Proteinlöslichkeit im Bereich von 30-60 %.

- Wasserbindung:

  Die Wasserbindung, bestimmt nach dem AACC-Bestimmungsverfahren, beträgt mindestens 2 ml/g, bevorzugt mindestens 3 ml/g. Vergleichsmessungen zeigen, dass die Wasserbindung des Präparats mindestens 30 % der Wasserbindung von Pisane®, bestimmt nach dem AACC-Bestimmungsverfahren, beträgt.

- Ölbindung:

  Die Ölbindung, bestimmt nach dem Fettbinde-Bestimmungsverfahren, beträgt mindestens 1 ml/g, bevorzugt mindestens 4 ml/g. Vergleichsmessungen zeigen, dass die Ölbindung mindestens 100% der Ölbindung von Pisane® oder von Supro® EX33, bestimmt nach demselben Verfahren, beträgt.

- Emulgierkapazität:

  Die Emulgierkapazität, bestimmt nach der Konduktivitätsmessungs-Methode, beträgt mindestens 400 ml Öl/g, bevorzugt mindestens 500 ml Öl/g. Vergleichsmessungen zeigen, dass die Emulgierkapazität mindestens 40% der Emulgierkapazität von Natriumkaseinat FN5S, bestimmt nach demselben Verfahren, beträgt.

- Schaumbildende Eigenschaften:

  • Schaumaktivität:

    Die Schaumaktivität beträgt mindestens 1000 %. Vergleichsmessungen mit frischem Hühnereiklar bei Aufschlag während 3 min auf Stufe 3 in einer Hobart 50N Standard-Küchenmaschine mit Rührbesen zeigen, dass die Schaumaktivität des Proteinpräparats mindestens 50 % oder sogar mindestens 60 % der Schaumaktivität von Hühnereiklar entspricht.

  • Schaumdichte:

    Die Schaumdichte liegt im Bereich von 80 und 110 g/l. Vergleichsmessungen mit Hühnereischnee nach Aufschlag während 3 min auf Stufe 3 in einer Hobart 50N Standard-Küchenmaschine mit Rührbesen zeigen, dass die Schaumdichte im Bereich von 80 und 110 % der Schaumdichte von Hühnereischnee liegt.

- Schaumstabilität:

    Die Schaumstabilität beträgt mindestens 80 %, vorzugsweise mindestens 90 %. Sie entspricht typischerweise mindestens 90 % der Schaumstabilität von Hühnereischnee, gemessen als Volumenabnahme von 100 ml Hühnereischnee innerhalb einer Stunde nach Aufschlag während 3 min auf Stufe 3 in einer Hobart 50N Standard-Küchenmaschine mit Rührbesen.

Sensorische Eigenschaften:

Nebst der hellen Farbe ist das Proteinpräparat, insbesondere in Form des SBPK, im Wesentlichen geruchsfrei und geschmacksneutral. Insbesondere fehlen im Wesentlichen die pflanzen- oder saateigenen Aromen. So sind im Wesentlichen kein bohniger und grasiger Geruch und Geschmack sowie im Wesentlichen kein Bittergeschmack wahrnehmbar.

Sensorische Tests, in welchen geschulte Prüfer einen bestimmten Geschmacks- oder Aromaeindruck des Proteinpräparats und einer geeigneten Referenzsubstanz vergleichen und auf einer Skala von 1 bis 10 (1 = nicht wahrnehmbar, 10 = stark wahrnehmbar) bewerten, wobei die Referenzsubstanz so gewählt ist, dass bei ihr der zu prüfende Geschmacks- oder Aromaeindruck mit mindestens 8 bewertet wird, zeigen, dass dem Proteinpräparat ein Wert von 3 oder weniger (typischerweise ein Wert von 1) zugeordnet wird.

Beispiele von zu testenden Geschmacks- oder Aromaeindrücken sind:

- bohniger Geschmack im Vergleich zu Sojabohnen;
- Grüner bis grasiger Geschmack im Vergleich zu grünem Paprika oder grünen Erbsen;
- Bittergeschmack im Vergleich zu einer 0,1 %igen wässrigen Koffeinlösung.

Farbe, Eigengeschmack und Eigengeruch des Proteinpräparats sind so, dass bei der Einarbeitung in Lebens- und Futtermittel im Wesentlichen keine mit üblichen statistischen Methoden ermittelte signifikante, als negativ zu wertende Veränderung des arteigenen Aussehens, Geruchs und Geschmacks der fertigen Zubereitung auftritt.

Sensorische Tests zeigen, dass die Geschmacks- und Aromaänderung, die in einer Lebensmittelzubereitung durch den Einsatz des Proteinpräparats hervorgerufen wird, gegenüber der Lebensmittelzubereitung ohne das Proteinpräparat auf ein derartiges Maß begrenzt ist, dass ein geschulter Prüfer eine Abweichung eines der oben genannten Geschmacks- oder Aromamerkmale auf einer Skala von 1-10 von maximal 3 Stufen, besser maximal 1 Stufe (fast nicht mehr erkennbare Abweichung) erkennen kann.

Bei dem vorgeschlagenen Verfahren wird durch die Anwendung von mit dem saateigenen Wasser vermischtem Alkohol oder wässrigen Alkohollösungen der Großteil der pflanzeneigenen Aromastoffe und weiteren sekundären Pflanzenstoffe wie Phenolsäuren entfernt. Dadurch werden helle, verfärbungsstabile und nahezu geruchs- und geschmacksneutrale Mehle erhalten.

Überraschenderweise kann der Proteingehalt des Raffinats/ Raffinats/Mehls auf diese Weise durch die Mitextraktion anderer niedermolekularer Bestandteile, insbesondere die enthaltenen Zucker, auf Anteile größer/gleich 60% erhöht werden, so dass ohne weitere Prozessschritte hochwertige stabile Proteinkonzentrate erhalten werden.

In der Alkohol-, wässrigen oder Wasser-AlkoholPhase fällt dabei ggf. ein Gemisch von Zuckern/ Oligosacchariden und sekundären Pflanzenstoffe wie Phenolsäuren an. Besonders vorteilhaft können die Zuckerstoffe als Trägerstoff für die phenolischen Stoffe z.B. bei einer anschließenden Trocknung zur Herstellung einer Anwendungsform dienen. Durch selektive Adsorption, Kristallisation oder Fällung können die beiden Fraktionen weiter gereinigt oder aufgetrennt werden, um beide Fraktionen getrennt nutzbar zu machen.

Die Erfinder haben außerdem erkannt, dass sich bei einer Extraktion mit $scCO_2$ besondere Vorteile ergeben bei der weiteren nasstechnischen Verarbeitung der entölten Schrote bzw. Mehle durch den verminderten Gehalt an störenden Begleitstoffen, weil das im Schrot enthaltene $CO_2$ gleichzeitig in einer nachfolgenden Verarbeitung stabilisierend wirkt, da Oxidationsprozesse eingeschränkt werden.

Ein weiterer Vorteil ergibt sich, wenn die gewonnenen Präparate nach der $scCO_2$-Behandlung direkt abgepackt werden. Überraschenderweise sind sie dann ohne weitere Schutzgaszugabe direkt vor Oxidation geschützt. Eine Teilbelüftung oder Kombination mit anderen Schutzgasen kann dennoch vorteilhaft sein.

Weiterhin wurde erkannt, dass die funktionellen Eigenschaften der Proteinpräparate durch die Einstellung der Korngröße modifiziert werden können. Durch eine entsprechende Feinzerkleinerung oder Fraktionierung nach Korngröße oder Korndichte des Sonnenblumenproteinmehls oder des Konzentrats können die Wasserbindung sowie die Emulgierkapazität gezielt eingestellt werden, um unterschiedliche Anforderungen zu erfüllen. Besonders vorteilhaft wird dabei eine Korngröße von $\leq 500\ \mu$m eingestellt oder eine Fraktion mit einer Korngröße $\leq 500\ \mu$m abgetrennt.

Nach dem erfindungsgemäßen Verfahren ist mit einem minimalen Einsatz von Wasser die Herstellung hochwertiger Sonnenblumenproteinpräparate möglich, die überraschenderweise ähnlich gute Eigenschaften wie Proteinisolate aufweisen, obwohl sie einen geringeren Proteingehalt haben. Mit Hilfe der beschriebenen Technologie werden Sonnenblumenkerne nahezu vollständig in ernährungsphysiologisch und technofunktionell wertvolle Lebensmittelinhaltsstoffe und weitere Fraktionen für eine energetische und technische Nutzung fraktioniert, wobei die Proteinausbeute besonders hoch ist.

So wurde auch erkannt, dass die anfallende alkoholische zuckerhaltige Lösung direkt für die Fermentation zu Bio-Ethanol verwendet werden kann.

Die Erfinder haben außerdem erkannt, dass das Öl, das durch die unpolare Extraktion gewonnen wird und Reste von Hexan enthält, ohne weitere Aufbereitung für die Beimischung zu oder Herstellung von Biodiesel geeignet ist oder direkt als Brennstoff genutzt werden kann.

In einer anderen vorteilhaften Ausgestaltung des Verfahrens werden die Extraktionen so hintereinander geschaltet, dass mit dem überkritischen $CO_2$ gleichzeitig der im Schrot gebundene Restalkohol extrahiert wird, so dass nachfolgende Destillations- oder Reinigungsschritte entfallen. Die Erfinder haben festgestellt, dass das Öl, das durch die unpolare Extraktion gewonnen wird und Reste von Alkohol enthält, sich erstaunlicherweise sehr gut zur Weiterverarbeitung zu Biodiesel eignet und in einem Prozess, der auf enzymatischer Umesterung von Fett mit Alkohol beruht, direkt eingesetzt werden kann. Daher werden besonders vorteilhaft eventuell in das Öl verschleppte Alkoholanteile nicht entfernt sondern bleiben enthalten und werden bei einer weiteren Verarbeitung des Öls zu Biodiesel nach dem Umesterungsverfahren genutzt.

**Ausführungsbeispiele**

**Beispiel 1: Sonnenblumenkernproteinkonzentrat aus alkoholischer Extraktion aus entölten, geschälten Sonnenblumenkernen**

Geschälte Speisekerne mit einer Reinheit von 99,9%, d.h. einem Schalenanteil von < 0,1%, wurden in einer Schneckenpress mit einer Düse von 5mm Durchmesser bei einer Temperatur von ca. 40°C (+-5°C) auf einen Restfettgehalt von 23 % entölt und die erhaltenen strangförmigen Presslinge in einem Soxhlet für 36h mit n-Hexan entölt und bei Raumtemperatur luftgetrocknet, um Hexanreste zu entfernen. Das so erhaltene Hexanentölte phenolsäurehaltige Sonnenblumenschrot aus geschälten Speisekernen wurde im Soxhlet mit Methanol (95%) extrahiert, wobei die Alkoholkonzentration sich durch Extraktion des Wassers von anfänglich ca. 80% (v/v) auf ca. 95% erhöhte. Die Temperatur des Ölbads betrug ca. 85 °C, die Extraktionstemperatur betrug zwischen 20 °C (Kühler) und maximal 65 °C (Siedetemp. Methanol = 65 °C). Nach 12 Durchläufen wurde die Extraktion beendet, nachdem schon mehrere Durchläufe keine gelbliche Färbung des Extrakts mehr erkennbar war.

Das so erhaltene Sonnenblumenproteinkonzentrat ist ein feines helles Pulver mit einem Proteingehalt von > 60 %. Die Zusammensetzung ist in nachfolgender Tabelle angegeben.

| Nr. | Probenbezeichnung | Trocken-masse (TS) | Protein in TS | Fett in TS (Büchi) | Asche in TS | Phenolsäuren in TS | Emulgierkapazität |
|---|---|---|---|---|---|---|---|
| | | % | % | % | % | % | ml/g |
| 1 | Sonnenblumenkernmehl (Schrot aus geschälten Sonnenblumenkernen) | 90,4 | 61,1 | 3,6 | 7,5 | 0,50 | 510 |
| 2 | Sonnenblumenproteinkonzentrat (aus 1) | 87,8 | 76,5 | 1,5 | 8,6 | 0,01 | 210 |
| 3 | Sonnenblumenproteinisolat (aus 2) | 89,1 | 96,2 | 0,29 | 4,20 | 0,00 | 675 |

Das Proteinkonzentrat ist arm an pflanzeneigenen Aromakomponenten. Die Farbe dieses schalenarmen Sonnenblumenproteinmehls und -konzentrats ist besonders ansprechend bzw. neutral und wird nach CIE L*a*b* mit folgenden Werten wiedergegeben:

| Nr. | | L* | a* | b* |
|---|---|---|---|---|
| 1 | Sonnenblumenkernmehl | 90,0 | 0,47 | 6,33 |
| 2 | Sonnenblumenproteinkonzentrat (aus1) | 86,8 | 0,18 | 8,11 |
| 3 | Sonnenblumenproteinisolat (aus 2) | 73,9 | 1,22 | 10,94 |

Das Sonnenblumenmehl enthielt vorher ca. 0,5 % Kaffeesäurederivate, nachgewiesen mit HPLC (elektrochemische Detektion) und quantifiziert mit photometrischer Bestimmung. Das extrahierte Sonnenblumenmehl, nachfolgend Sonnenblumenproteinkonzentrat genannt, enthielt nur noch Spuren von Chlorogensäure, d.h. an der Nachweisgrenze von 0,01%. Es wurden demnach 90% der Phenolsäuren extrahiert, identifiziert und quantifiziert als Kaffeesäurederivate. Die extrahierte Menge wurde vollständig im Extrakt wiedergefunden. Der Trockenmasseverlust (TS) betrug 24%. Die extrahierte Trockenmasse bestand zu geringen Anteilen aus Protein, Fett und Mineralstoffen. Neben Phenolsäuren waren hauptsächlich Zucker, Oligosaccharide, Ballaststoffe enthalten, die insgesamt zu 63% extrahiert wurden, wovon Oligosaccharide wie Raffinose bei vollständiger Extraktion maximal 30% ausmachen. Überraschenderweise gehen also andere sekundäre Pflanzenstoffe, insbesondere Phytinsäure in den Extrakt über.

Die Phenolsäuren wurden fast vollständig aus dem Schrot extrahiert und konnten im Extrakt nachgewiesen werden. Der Mineralstoffgehalt des Schrotes nahm durch die Behandlung mit Methanol geringfügig zu, während übrige Begleitstoffe entfernt wurden. Die Extraktion mit Methanol führt zu einer weitgehenden Abreicherung störender Begleitstoffe, insbesondere der Phenolsäuren sowie von Ölbegleitstoffen. Der Proteingehalt wurde auf über 60% erhöht, so dass ein farbstabiles Proteinkonzentrat gewonnen werden kann oder eine folgende nasstechnische Gewinnung hochwertiger Proteinisolate nicht durch Polyphenole gestört wird (siehe Farbe des Proteinisolats, Tabelle).

**Beispiel 2: Pressen von geschälten Sonnenblumenkernen**

Geschälte Speisekerne wurden mit einer Schneckenpresse bei 40 - 50°C mit 3 unterschiedlichen Düsen entölt, die jeweils einen Durchmesser von 6, 5 und 4 mm haben. Die erhaltenen Presskuchen unterschieden sich im Fettgehalt sowie in ihrer Struktur und Farbe (Tabelle 2-1). Der Fettgehalt wurde nach zwei Methoden bestimmt, wobei die Büchi-Methode (nach Caviezel) den Gesamt-Fettgehalt angibt und die Soxtherm-Methode den extrahierbaren Anteil bestimmt.

Tabelle 2-1

| Probenbezeichnung | Trockenmasse (TS) | Protein in TS | Fett in TS (Büchi) | Fett in TS (Soxtherm) |
|---|---|---|---|---|
| | % | % | % | % |
| geschälte Sonnenblumenkerne | 94,7 | 25,2 | 55,9 | 52,2 |
| Presskuchen 1 Düse 6mm | 92,4 | 37,9 | 36,3 | 34,5 |
| Presskuchen 2 Düse 5mm | 92.3 | 37,9 | 35,3 | 33,4 |
| Presskuchen 3 Düse 4mm | 90,3 | 52,8 | 9,6 | 6,6 |

Beim Pressen mit der dünnsten Düse erhöhte sich der Druck in der Presse deutlich, so dass ein sehr fester Presskuchen mit geringem Fettgehalt von ca. 10% erreicht wurde. Der Presskuchen war unter diesen Bedingungen allerdings dunkler, was auf Oxidation oder Maillardreaktion schließen lässt. Bei der schonenderen Pressung mit einer größeren Düse 5/6 mm war der Restfettgehalt mit ca. 33 bzw. 35% wesentlich höher. Allerdings konnte dieser durch nachfolgende Extraktion auf unter 1 % abgesenkt werden (Tabelle 2-1).

Die Bestimmung der funktionellen Eigenschaften ergab eine Verbesserung der Proteinlöslichkeit sowie der Emulgierkapazität gegenüber den Ausgangskernen bei den letzten beiden Presskuchen, d.h. dass ein verbesserter Zellaufschluss und eine gute Porosität durch das Pressen erreicht wurden. Die Porosität geht mit zunehmendem Pressgrad verloren. Um eine optimale Festigkeit des Presskuchens mit einer guten mechanischen Stabilität während der nachfolgenden Extraktion zu erhalten, sollte der Pressgrad dagegen möglichst hoch sein.

Neben der Düsengeometrie wirkte sich auch die Temperatur der Pressung auf den Entölungsgrad und die Struktur der Presslinge aus. Die Festigkeit von Presslingen mit rundem Querschnitt wurde mittels Texture Analyzer (TA) bei einer radialen Druckbeanspruchung mit einem Stempel mit 75 mm Durchmesser bei einer Stempelgeschwindigkeit von 1mm/s bestimmt. Gemessen wurde die maximale Kraft, die aufgewendet wurde bis zum Bruch des Presslings. Die Kraft wurde bezogen auf die beanspruchte Fläche von 1mm Breite und der Länge des Presslings unter dem Stempel Der Mittelwert des Bruchdrucks wurde anhand von jeweils 20 Proben bestimmt.

Tabelle 2-2

| | Fettgehalt | Mittelwert Bruchdruck [N/mm2] | Schüttdichte [kg/m$^3$] |
|---|---|---|---|
| Düse 5mm, gepresst bei < 40°C | 35% | 0,58 | 310 |
| Düse 5mm gepresst bei < 40°C | 23% | 0,96 | 350 |
| Düse 5mm, gepresst bei 55°-60°C | 17% | 4,81 | 420 |
| Düse 8mm, gepresst bei 60-70°C | 11 % | 9,76 | 470 |

Ein sehr niedriger Restfettgehalt von 11% konnte auch mit einer größeren Düse mit 8 mm Durchmesser (Tabelle 2-2) erzielt werden, wenn die Temperatur auf bis zu 70°C erhöht wurde. Diese Presslinge wiesen eine sehr hohe mechanische Stabilität auf, hatten jedoch eine hohe Schüttdichte und daher niedrigere Porosität und wiesen eine etwas dunklere Farbe auf als die Presskuchen, die bei 40°C erhalten wurden. Bei Anwendung von niedrigeren Temperaturen unter 60°C konnte ein annähernd festes Material mit jedoch deutlich höherer Porosität erhalten werden (Tabelle 2-2), dass nur wenig thermische Schädigung aufwies und sich sehr gut extrahieren ließ. Dagegen wurden bei Temperaturen > 70°C bereits deutliche Proteinschäden und eine starke Verfärbung festgestellt.

Es wurde erkannt, dass bei einem Pressgrad auf einen Restfettgehalt im Bereich von ca. 15 % bis 25 % Restfett, Presskuchen-Pellets erhalten werden, die trotz fehlender Schalen eine gute mechanische Stabilität bei noch ausreichend vorhandener Porosität aufweisen, so dass ohne weitere Strukturierung oder Zerkleinerung bei der nachfolgenden Extraktion eine vollständige Entölung möglich ist. Überraschenderweise weisen die Pellets auch nach der Entölung trotz der mit der Entfernung des Öls einhergehenden Lockerung der Struktur noch eine ausreichende mechanische Stabilität auf, so dass sie einer Extraktion mit einem weiteren Lösemittel unterworfen und so an proteinfremden Substanzen abgereichert werden können, ohne auseinander zu fallen. Durch die poröse Struktur haben sie ein sehr günstiges Extraktionsverhalten für eine weitere Extraktion, z.B. mit alkoholischer Lösung. Dadurch kann auf eine Strukturierung oder Zerkleinerung vor der Extraktion, die sonst üblicherweise durchgeführt werden muss, verzichtet werden.

Je nach Konfiguration und Geometrie der Pressvorrichtung ist der optimale Pressgrad bei einem Restölgehalt von ca. 15% bis 25 % erreicht (vgl. Fig. 2). Dabei wurde erkannt, dass auch bei Temperaturen unter 60°C eine ausreichende Verdichtung der Presslinge erzielt wird und gleichzeitig die Funktionalität und Farbe der Proteine am besten erhalten bleiben.

Insgesamt können durch ein Pressen, bei welchem ein bestimmter Restfettgehalt bestehen bleibt, die Partikel so strukturiert werden, dass eine nachträgliche Strukturierung oder Zerkleinerung nicht mehr erforderlich ist, die sonst üblicherweise zum Aufbrechen des Presskuchens vor der Extraktion durchgeführt wird. Neben der Vereinfachung des Verfahrens trägt dies zur Schonung des Presskuchens bei, so dass u. a. Proteinfunktionalität und die Farbe im Endprodukt verbesserbar sind.

Auch werden durch den reduzierten Pressgrad die Proteine geschont und die funktionellen Eigenschaften des Proteinpräparats bleiben in verbessertem Masse erhalten. Dabei wird gleichzeitig eine Partikelform hergestellt, die eine optimale Extraktion ermöglicht und so der Restölgehalt nach dem Entölen weiter gesenkt werden kann. Auch dies trägt u. a. zu einer Verbesserung der Farbe des Proteinpräparats bei.

**Beispiel 3: Sonnenblumenkernmehle und Proteinkonzentrate, erhalten durch Entölung und Extraktion von schalenfreien Presskuchen mit Hexan, scCO2 und Ethanol**

Herstellung:

1. Schälung der Sonnenblumensamen und Auftrennung in eine Kern- und eine Schalenfraktion und Verwendung der Kernfraktion mit einem Schalengehalt von maximal 0,5% (w/w)
2. mechanische Teilentölung auf einen Restfettgehalt von ca. 36% durch Pressung, wie bei Beispiel 2
3. Entölung des Sonnenblumenpresskuchens a) mit iso-Hexan in einem Perkolator bei Temperaturen von maximal 60°C oder b) Extraktion mit überkritischem $CO_2$ in einem Druckbehälter (Einstellungen s.u., Tabelle)
4. Extraktion des Presskuchens aus 2 oder des Proteinmehls aus 3a mit Ethanol und/oder Hexan in einer Soxhlet-Apparatur (Einstellungen s.u., Tabelle)

5. Austreiben des Hexans nach der Extraktion 3a mit überhitztem Hexandampf unter Vakuum (< 500 hPa)

6. Austreiben weiteren Hexans aus 5 mit überhitztem Wasserdampf unter Vakuum (< 500 hPa).

7. Entfernung von Lösemittelresten aus 6 durch Erhitzung auf 60 °C im Vakuum (< 500 hPa). Das so erhaltene Raffinat wird nachfolgend Proteinmehl genannt.

8. Entfernung des Lösemittels nach Extraktionen 4 im Luftstrom bei Raumtemperatur, um Proteinkonzentrate zu erhalten.

9. Verdampfung des Alkohols und Trocknung des im Verfahrensschritt 8 erhaltenen Raffinats im Rotationsverdampfer unter Vakuum bei maximal 50°C, um ein Sonnenblumenproteinkonzentrat zu erhalten.

10. Vermahlung der Sonnenblumenproteinmehle und konzentrate aus Schritt 3b, 7 oder 9 in einer Stiftmühle mit Siebeinsatz 0,5 mm, um die Sonnenblumenproteinpräparate als feines Pulver zu erhalten.

11. Einsatz der Proteinmehle und Proteinkonzentrate mit oder ohne vorherige oder nachfolgende Zerkleinerung.

Die Pellets aus der Schneckenpresse (5-mm-Düse aus Beispiel 2) wurden anschließend auf zwei unterschiedliche Arten entölt, 1. mit Hexan (Entölung und Desolventierung bei Temperaturen unterhalb 60°C) und 2. mit überkritischem $CO_2$. Dabei wurde mit Hexan eine vollständige Entölung erzielt, die Extraktion mit $CO_2$ bei $800*10^5$ Pa war ebenfalls nahezu vollständig, bei $285*10^5$ Pa wurde dagegen knapp 20 % weniger Öl extrahiert (50°C, 100 kg/kg $CO_2$). Die Untersuchung der Säurezahlen der Öle aus beiden Extraktionsverfahren ergab keine grundlegenden Unterschiede. Hiermit wurde außerdem gezeigt, dass sich die Pellets ohne weitere Zerkleinerung oder Aufbereitung sehr gut zur Extraktion eignen.

Tabelle 3-1

| Nr. | Probenbezeichnung und Herstellung | Trockenmasse (TS) | Protein in TS (Nx6,25) | Fett in TS (Büchi) | Asche in TS | EC |
|---|---|---|---|---|---|---|
| | | % | % | % | % | |
| 1 | Presskuchen | 92,3 | 42,3 | 35,3 | 4,6 | 505 |
| 2 | Sonnenblumenkernmehl Hexan-entölt <60°C (aus 1) | 90,8 | 63,6 | 3,0 | 7,7 | 510 |
| 3 | Sonnenblumenkernmehl scCO2-extrahiert 50°C, $285*10^5$ Pa (aus 1) | 94,2 | 58,6 | 10,7 | 6,7 | 598 |
| 4 | Sonnenblumenkernmehl scCO2-extrahiert 50°C, $800*10^5$ Pa (aus 1) | 93,6 | 62,7 | 3,8 | 7,7 | 513 |
| 5 | Sonnenblumenkernproteinkonzentrat mit Ethanol extrahiert (aus 1) | 92,4 | 58,9 | 15,7 | 6,9 | 280 |
| 6 | Sonnenblumenkernproteinkonzentrat mit Ethanol extrahiert (aus 2) | 90,9 | 69,0 | 0,4 | 8,4 | 380 |
| 7 | Sonnenblumenkernproteinkonzentrat mit Ethanol extrahiert und mit Hexan entölt, fließender Übergang (aus 5) | 90,4 | 70,3 | 0,3 | 8,4 | 285 |
| 8 | Sonnenblumenkernproteinkonzentrat mit Ethanol extrahiert, getrocknet und anschließend mit Hexan entölt (aus 5) | 90,3 | 69,0 | 0,2 | 8,2 | 285 |

Eigenschaften:

Die so erhaltenen Sonnenblumenproteinmehle und - konzentrate haben einen Proteingehalt von mindestens 50% (N x 5,6) und eine weitere Zusammensetzung sowie funktionelle Eigenschaften, wie in nachfolgender Tabelle angegeben. Die so erhaltenen Sonnenblumenproteinkonzentrate (Nr.6-8) sind frei von sonnenblumeneigenen Aromakomponenten. Das Mehl (Nr. 2) wies noch einen gewissen sonnenblumen-nussigen Eigengeschmack auf. Nach einfacher Vermahlung und Siebung (<263 mm) wurde es zur Emulgierung einer Ei-freien Salatmayonnaise eingesetzt, die vergleichbar homogen und stabil war wie mit einem Pflanzenproteinisolat und sensorisch gut beurteilt wurde.

Die Farbe des schalenfreien Sonnenblumenproteinmehls und der Sonnenblumenproteinkonzentrate ist besonders ansprechend, d. h. neutral und weist nach CIE-L*a*b* folgende Werte auf:

Tabelle 3-2

| Nr. | Probenbezeichnung und Herstellung | L* | a* | b* |
|---|---|---|---|---|
| | dieselben Präparate aus Tabelle 3-1 | % | % | % |
| 1 | Presskuchen | 70,5 | 1,95 | 12,75 |
| 2 | Sonnenblumenkernmehl Hexan-entölt <60°C (aus 1) | 89,21 | 0,59 | 6,48 |
| 3 | Sonnenblumenkernmehl scCO2-extrahiert 50°C, 285*10$^5$ Pa (aus 1) | 88,4 | 0,49 | 7,49 |
| 4 | Sonnenblumenkernmehl scCO2-extrahiert 50°C, 800*10$^5$ Pa (aus 1) | 89,3 | 0,31 | 6,79 |
| 5 | Sonnenblumenkernproteinkonzentrat mit Ethanol extrahiert (aus 2) | 88,0 | -0,0 | +7,8 |

**Beispiel 4: Sonnenblumenkernproteinmehle aus entölten, geschälten Sonnenblumenkernen mit modifizierten Eigenschaften durch Einstellung der Korngröße**

Bei diesem Beispiel wurde unter anderem die Modifizierung der funktionellen Eigenschaften des Sonnenblumenproteinpräparats untersucht bei einer abschließenden Aufbereitung in der Korngröße.

Herstellung:

1. Schälung der Sonnenblumensamen und Auftrennung in eine Kern- und eine Schalenfraktion
2. mechanische Teilentölung auf einen Restfettgehalt von ca. 36% durch Pressung, siehe Beispiel 2
3. Entölung des Sonnenblumenpresskuchens mit iso-Hexan in einem Perkolator bei Temperaturen von maximal 60°C
4. Austreiben des Hexans mit überhitztem Hexandampf unter Vakuum (< 500 hPa)
5. Austreiben weiteren Hexans mit überhitztem Wasserdampf unter Vakuum (< 500 hPa).
6. Entfernung von Lösemittelresten durch Erhitzung auf 60 °C im Vakuum (< 500 hPa). Das so erhaltene Raffinat wird nachfolgend Proteinmehl genannt.
7. Sichtung, Siebung und/oder Vermahlung des Proteinkonzentrats in einer Stift- oder Schlagmühle, um Fraktionen mit unterschiedlicher Partikelgrößenverteilung zu erhalten und so die funktionellen Eigenschaften zu modifizieren
8. Einsatz der Proteinmehle und Proteinkonzentrate mit oder ohne vorherige oder nachfolgende Zerkleinerung.

Das entölte Mehl (Nr. 2) wurde nur gesiebt (< 263 mm) und direkt zur Emulgierung einer Ei-freien Salatmayonnaise eingesetzt, die vergleichbar homogen und stabil war wie mit einem Pflanzenproteinisolat. Der Geschmack und die Textur konnten weiter verbessert werden, wenn das Proteinmehl vermahlen wurde.

Durch eine Aufbereitung in der Korngrösse, wie sie oben im letzten Schritt 7 durchgeführt wurde, waren die funktionellen Eigenschaften der Sonnenblumenkernproteinpräparate veränderbar. Zur Korngrössenverkleinerung wurde neben einer reinen Vermahlung auch eine Sichtung oder eine Siebung, gegebenenfalls in Verbindung mit einer Vermahlung eingesetzt. Mit abnehmender Korngrösse nahm die Wasserbindung tendenziell zu, beim SBK ebenso die Emulgierkapazität, während die Ölbindung geringfügig abnahm oder sich kaum änderte. Die Präparate mit einer homogeneren Korngrössenverteilung haben eine höhere Wasserbindung. Als besonders vorteilhaft zur Steigerung der Wasserbindung stellte sich die Kombination von Fraktionierung und Zerkleinerung heraus. Insgesamt ist es möglich, das funktionelle Profil durch eine zielgerichtete Aufbereitung in der Korngrößenverteilung zu modifizieren.

Die Verfahrensmerkmale der Ansprüche 1 oder 2 lassen sich noch kombinieren mit:

- einem Verfahren bei dem die Temperatur der geschälten Sonnenblumensamen bei der Teilentölung und weiteren Entölung in einem Bereich zwischen 10°C und 80°C gehalten wird; bevorzugt bei ≤ 70°C, besonders bevorzugt bei ≤ 60°C, gehalten wird.

**Patentansprüche**

1. Verfahren zur Gewinnung von Proteinpräparaten aus Sonnenblumensamen mit wenigstens folgenden Schritten:

- Schälen der Sonnenblumensamen bis auf einen Restschalengehalt von ≤ 5 Gew.% oder Bereitstellen von geschälten Sonnenblumensamen mit einem Restschalengehalt von ≤ 5 Gew.%,
- mechanische Teilentölung der geschälten Sonnenblumensamen durch Pressen, dabei Kontrolle der Temperatur auf unter 80°C, wobei zur Pressung eine Schneckenpresse oder ein Extruder verwendet wird,
- Durchführung eines oder mehrerer Extraktionsschritte mit mindestens einem Lösungsmittel, durch die ein entfettetes proteinhaltiges Mehl als Proteinpräparat erhalten wird, wobei mindestens einer der Extraktionsschritte eine weitere Entölung der teilentölten geschälten Sonnenblumensamen bewirkt,
- wobei die mechanische Teilentölung bis auf einen Fett- oder Ölgehalt der geschälten Sonnenblumensamen im Bereich zwischen 10 und 35 Gew.% durchgeführt wird, bei dem durch das Pressen mit der Schneckenpresse oder dem Extruder ein Presskuchen in der Form von Pellets oder Strängen erhalten wird, der eine Dicke im Bereich zwischen 0,2 und 4 cm aufweist, und
- wobei der mindestens eine Extraktionsschritt am Presskuchen in der Form von Pellets oder Strängen durchgeführt wird.

2. Verfahren nach Anspruch 1,
bei dem die Teilentölung bis auf einen Fett- oder Ölgehalt der geschälten Sonnenblumensamen im Bereich zwischen 12 und 25 Gew.%, bevorzugt im Bereich zwischen 17 und 25 Gew.%, durchgeführt wird und/oder bei dem die Teilentölung Presskuchen in Form von Strängen mit annähernd rundem Querschnitt mit einem Durchmesser zwischen 0,4 und 4 cm, vorzugsweise im Bereich zwischen 0,5 und 2 cm, oder einem eckigen Querschnitt mit einer Kantenlänge zwischen 0,4 und 4 cm, vorzugsweise im Bereich zwischen 0,5 und 2 cm, in mindestens einer Dimension ergibt, die eine Bruchfestigkeit von 2 - 10 N/mm$^2$, besser 4 - 8 N/mm$^2$ aufweisen.

3. Verfahren nach Anspruch 1 oder 2,
bei dem die Temperatur der geschälten Sonnenblumensamen bei der Teilentölung und weiteren Entölung in einem Bereich zwischen 10°C und 80°C, bevorzugt bei ≤ 70°C, besonders bevorzugt bei ≤ 60°C, gehalten wird.

4. Verfahren nach einem der Ansprüche 1 bis 3,
bei dem die Teilentölung und der eine oder die mehreren Extraktionsschritte so durchgeführt werden, dass ein Denaturierungsgrad der Proteine im entfetteten proteinhaltigen Mehl maximal 40%, vorzugsweise zwischen 10% und 30%, beträgt.

5. Verfahren nach einem der Ansprüche 1 bis 4,
bei dem die mehreren Extraktionsschritte eine Kombination von mindestens einem lipophilen Extraktionsschritt mit einem lipophilen Lösungsmittel und mindestens einem hydrophilen Extraktionsschritt mit einem hydrophilen Lösungsmittel umfassen.

6. Verfahren nach Anspruch 5,
bei dem durch den hydrophilen Extraktionsschritt proteinfremde Stoffe aus dem proteinhaltigen Mehl abgereichert werden und Proteine weitgehend (d.h. mit maximal 10% Proteinverlust) ungelöst bleiben, wobei als hydrophiles Lösungsmittel ein wässrigalkoholisches Lösungsmittel mit einem Alkoholanteil zwischen 600 und 800 Milliliter Alkohol pro Liter des Lösungsmittels eingesetzt wird.

7. Verfahren nach Anspruch 5 oder 6,
bei dem mehrere hydrophile Extraktionsschritte mit einem alkoholhaltigen Lösungsmittel durchgeführt werden, wobei bei mindestens einem Übergang von einem zum nächsten hydrophilen Extraktionsschritt der Alkoholgehalt im alkoholhaltigen Lösungsmittel erhöht wird.

8. Verfahren nach Anspruch 7,
bei dem ein Restwassergehalt nach dem ersten hydrophilen Extraktionsschritt 20 - 30 % beträgt und dieser durch eine weitere Extraktion mit einem hydrophilen Lösungsmittel auf 5 -10% gesenkt wird.

9. Verfahren nach einem der Ansprüche 1 bis 4,
bei dem die Durchführung des Extraktionsschrittes mit Hexan als Lösungsmittel erfolgt.

10. Verfahren nach einem der Ansprüche 1 bis 4,
bei dem die Durchführung des Extraktionsschrittes mit scCO$_2$ als Lösungsmittel erfolgt.

11. Verfahren nach einem der Ansprüche 1 bis 10,

bei dem das proteinhaltige Mehl in der Korngröße verändert oder nach Korngröße oder Korndichte getrennt wird, um funktionelle Eigenschaften des proteinhaltigen Mehls zu modifizieren.

12. Verfahren nach Anspruch 11,
    bei dem die Korngröße des proteinhaltigen Mehls auf ≤ 500 μm eingestellt wird.

13. Verfahren nach einem der Ansprüche 1 bis 12,
    bei dem im Extraktionsrückstand verbliebene Lösungsmittel durch Anwendung von Vakuum bei Temperaturen unter 70°C, bevorzugt bei einem Vakuum von 200 - 800 hPa und einer Temperatur von 40 - 65 °C, entfernt werden.

**Claims**

1. Method for obtaining protein preparations from sunflower seeds
   having at least the following steps:

   - dehulling the sunflower seeds until a residual hull content ≤ 5% by weight remains, or providing dehulled sunflower seeds with a residual hull content of ≤ 5% by weight,
   - partial deoiling of the dehulled sunflower seeds mechanically, by pressing, controlling the temperature to under 80°C, wherein a screw press or an extruder is used for pressing,
   - carrying out of one or more extraction steps using at least one solvent to obtain a defatted, protein-containing flour as a protein preparation, wherein at least one of the extraction steps causes further deoiling of the partially deoiled dehulled sunflower seeds,
   - wherein the mechanical partial deoiling is carried out until a fat or oil content in the dehulled sunflower seeds in a range from 10 to 35% by weight is obtained, in which a press cake in the form of pellets or strands with a thickness in a range between 0.2 and 4 cm is obtained by said pressing with the screw press or the extruder, and
   - wherein the at least one extraction step is carried out on the press cake in the form of pellets or strands.

2. Method according to claim 1,
   in which the partial deoiling is carried out until a fat or oil content in the dehulled sunflower seeds in a range from 12 to 25% by weight, preferably in the range between 17 and 25% by weight is obtained, and/or in which the partial deoiling produces press cakes in the form of strands having an approximately round cross section with a diameter between 0.4 and 4 cm, preferably in the range between 0.5 and 2 cm, or having an angular cross section with an edge length between 0.4 and 4 cm, preferably in the range between 0.5 and 2 cm in at least one dimension, and which have a breaking strength of 2-10 N/mm$^2$, preferably 4-8 N/mm$^2$.

3. Method according to claim 1 or 2,
   in which the temperature of the dehulled sunflower seeds is maintained in a range between 10 °C and 80 °C, preferably ≤70 °C, particularly preferably ≤60 °C during the partial deoiling and further deoiling.

4. Method according to any one of claims 1 to 3,
   in which the partial deoiling and the one or more extraction steps are carried out in such manner that the degree of denaturing of the proteins in the defatted protein-containing flour is not more than 40%, preferably between 10% and 30%.

5. Method according to any one of claims 1 to 4,
   in which the multiple extraction steps comprise a combination of at least one lipophilic extraction step with a lipophilic solvent and at least one hydrophilic extraction step with a hydrophilic solvent.

6. Method according to claim 5,
   in which non-protein substances are removed from the protein-containing flour and proteins remain largely undissolved (i.e. with a maximum 10% protein loss) due to the hydrophilic extraction step, wherein an aqueous-alcoholic solvent with an alcohol portion between 600 and 800 millilitres of alcohol per litre of solvent is used as the hydrophilic solvent.

7. Method according to claim 5 or 6,
   in which multiple hydrophilic extraction steps with an alcohol-containing solvent are carried out, wherein the alcohol content in the alcohol-containing solvent is increased in at least one transition from one hydrophilic extraction step to the next.

8. Method according to claim 7,
   in which a residual water content is 20-30% after the first hydrophilic extraction step and this is reduced to 5-10% by a further extraction with a hydrophilic solvent.

9. Method according to any one of claims 1 to 4,
   in which the extraction step is carried out using hexane as the solvent.

10. Method according to any one of claims 1 to 4,
    in which the extraction step is carried out using $scCO_2$ as the solvent.

11. Method according to any one of claims 1 to 10,
    in which the grain size of the protein-containing flour is changed or the flour is separated according to grain size or grain density in order to modify functional properties of the protein-containing flour.

12. Method according to claim 11,
    in which the grain size of the protein-containing flour is adjusted to $\leq 500$ $\mu$m.

13. Method according to any one of claims 1 to 12,
    in which solvents remaining in the extraction residue are removed by applying a vacuum at temperatures below 70 °C, preferably at a vacuum from 200 - 800 hPa and a temperature from 40 - 65 °C.

**Revendications**

1. Procédé d'extraction de préparations de protéines à partir de graines de tournesol, comportant au moins les étapes suivantes :

   - peler les graines de tournesol jusqu'à une teneur de coque résiduelle de $\leq 5$ % en poids ou fournir des graines de tournesol pelées avec une teneur de coque résiduelle de $\leq 5$ % en poids,
   - déshuiler mécaniquement les graines de tournesol pelées par pressage, en contrôlant la température au-dessous de 80 °C, dans lequel une presse à vis ou une extrudeuse est utilisée pour le pressage,
   - effectuer une ou plusieurs étapes d'extraction avec au moins un solvant, par l'intermédiaire duquel une farine contenant des protéines dégraissées est obtenue comme préparation de protéines, dans lequel au moins une des étapes d'extraction provoque un déshuilage supplémentaire des graines de tournesol pelées déshuilées,
   - dans lequel le déshuilage mécanique est effectué jusqu'à une teneur en huile ou en graisse des graines de tournesol pelées dans la plage entre 10 et 35 % en poids, dans lequel on obtient par pressage avec la presse à vis ou l'extrudeuse un tourteau sous la forme de granules ou de fils, qui présente une épaisseur dans la plage entre 0,2 et 4 cm, et
   - dans lequel au moins une étape d'extraction est effectuée sur le tourteau sous forme de granules ou de fils.

2. Procédé selon la revendication 1,
   dans lequel le déshuilage est effectué jusqu'à une teneur en graisse ou en huile des graines de tournesol pelées dans la plage entre 12 et 25% en poids, de préférence dans la plage entre 17 et 25% en poids et/ou dans lequel le déshuilage produit un tourteau sous forme de fils avec une section transversale approximativement ronde avec un diamètre entre 0,4 et 4 cm, de préférence dans la plage entre 0,5 et 2 cm, ou une section transversale carrée avec une longueur d'arête entre 0,4 et 4 cm, de préférence dans la plage entre 0,5 et 2 cm, dans au moins une dimensions, qui présentent une résistance à la rupture de 2-10 N/mm$^2$, au mieux de 4-8 N/mm$^2$.

3. Procédé selon la revendication 1 ou 2,
   dans lequel la température des graines de tournesol pelées lors du déshuilage et du déshuilage supplémentaire est maintenue dans une plage entre 10°C et 80°C, de préférence à $\leq 70°C$, de manière encore préférée $\leq 60°C$.

4. Procédé selon une des revendications 1 à 3,
   dans lequel le déshuilage et la ou les étapes d'extraction supplémentaires sont effectuées de telle sorte qu'un taux de dénaturation des protéines dans la farine contenant des protéines dégraissée s'élève au maximum à 40%, de préférence soit compris entre 10% et 30%.

**5.** Procédé selon une des revendications 1 à 4,
dans lequel plusieurs étapes d'extraction comprennent une combinaison d'au moins une étape d'extraction lipophile avec un solvant lipophile et au moins une étape d'extraction hydrophile avec un solvant hydrophile.

**6.** Procédé selon la revendication 5,
dans lequel les substances étrangères aux protéines sont retirées de la farine contenant des protéines par l'intermédiaire de l'étape d'extraction hydrophile et les protéines restent essentiellement non dissoutes (c'est-à-dire, avec une perte de protéines maximale de 10%), dans lequel comme solvant hydrophile on utilise un solvant aqueux-alcoolique avec une fraction d'alcool comprise entre 600 et 800 millimètres d'alcool par litre de solvant.

**7.** Procédé selon la revendication 5 ou 6,
dans lequel plusieurs étapes d'extraction hydrophiles sont effectuées avec un solvant contenant de l'alcool, dans lequel lors d'au moins une transition d'une étape d'extraction hydrophile à la suivante la teneur en alcool dans le solvant contenant de l'alcool est augmentée.

**8.** Procédé selon la revendication 7,
dans lequel une teneur en eau résiduelle après la première étape d'extraction hydrophile s'élève à 20-30% et celle-ci est diminuée à 5-10% par une extraction supplémentaire avec un solvant hydrophile.

**9.** Procédé selon une des revendications 1 à 4,
dans lequel la mise en œuvre de l'étape d'extraction a lieu avec de l'hexane comme solvant.

**10.** Procédé selon une des revendications 1 à 4,
dans lequel la mise en œuvre de l'étape d'extraction a lieu avec du $scCO_2$ comme solvant.

**11.** Procédé selon une des revendications 1 à 10,
dans lequel la farine contenant des protéines est modifiée en ce qui concerne la taille de grain ou est séparée selon la taille ou l'épaisseur de grain, afin de modifier les propriétés fonctionnels de la farine contenant des protéines.

**12.** Procédé selon la revendication 11,
dans lequel la taille de grain de la farine contenant des protéines est réglée à $\leq 500$ $\mu$m.

**13.** Procédé selon une des revendications 1 à 12,
dans lequel le solvant resté dans le résidu d'extraction, est éliminé en utilisant du vide à des températures inférieures à 70°C, de préférence en présence d'un vide de 200-800 hPa et une température de 40-65°C.

Sonnenblumensamen

```
┌─────────────────────┐
│      Schälen        │ ─────────────→ Schalen   Energiegewinnung
└─────────────────────┘                           (Festbrennstoff), Bau-
           │                                      /Isoliermatieral
           ▼
┌─────────────────────┐
│    Konditionieren   │
└─────────────────────┘
           │
           ▼
┌─────────────────────┐
│       Pressen       │ ─────────────→ Öl   Nahrungsmittel,
└─────────────────────┘                      Biodiesel
           │
           ▼
┌─────────────────────┐
│ integrierte Extraktion │ ───────────→ Öl
│  (scCO₂ , Wasser    │
│  und/oder Alkohol)  │ ───────── Gemisch Sekundärer Pflanzenstoffe
└─────────────────────┘
           │                                          ▼
           ▼                              ┌─────────────────────┐
   Sonnenblumenmehl                       │    Stabilisierung   │
           │                              └─────────────────────┘
           ▼                                          │
┌─────────────────────┐                               ▼
│     Vermahlung      │                   ┌─────────────────────┐
│    / Klassierung    │                   │   Aufkonzentrierung │
└─────────────────────┘                   │     / Trocknung     │
           │                              └─────────────────────┘
           ▼                                          │
                                                      ▼
```

Gemisch Sekundärer Pflanzenstoffe (integrierte Extraktion $scCO_2$)

Proteinkonzentrat                          Konzentrat sekundärer Pflanzenstoffe
*Lebensmittel, Futtermittel,*
*Kosmetik, technische Applikation*         *Nahrungsmittel, Arzneimittel, Kosmetik,*
                                           *technische Applikation*

## Fig. 1

Extrahierbarkeit

Funktionalität

Festigkeit

0    10    20    30    40    50    60
Fettgehalt (nach dem Pressen), %

## Fig. 2

Sonnenblumensamen

↓

| Schälen | ——————→ Schalen |

↓

| Pressen | ——————→ Öl |

↓

| Entölung (Hexan<br>o. scCO$_2$ o. Alkohol) | ——————→ Öl |

↓

Sonnenblumenmehl

↓

| Extraktion<br>(Alkohol+Wasser) | ——————— Gemisch sekundärer Pflanzenstoffe |

↓

Sonnenblumen-<br>Proteinkonzentrat

↓

| Vermahlung<br>/ Klassierung |

↓

Proteinkonzentrat

Gemisch sekundärer Pflanzenstoffe

↓

| Stabilisierung |

↓

| Aufkonzentrierung<br>/ Trocknung |

↓

Konzentrat sekundärer Pflanzenstoffe

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

• WO 02060273 A1

**In der Beschreibung aufgeführte Nicht-Patentliteratur**

• **HECTOR BOURGES R. et al.** *Archivos latinoamericanos de nutrición,* 1980, vol. XXX (4), 564-579
• **MOHAMMAD SAEED ; MUNIR CHERYAN.** Sunflower Protein Concentrates and Isolates Low in Polyphenols and Phytate. *Journal of Food Science,* 1998, vol. 53 (4), 1127-1131
• **B. GASSMANN.** Preparation and application of vegetable proteins, especially proteins from sunflower seed, for human consumption. An approach. *Die Nahrung,* 1983, vol. 27 (4), 351-369
• **L. TRANCHINO et al.** Almost Complete Dehulling of High Oil Sunflower Seed. *JAOCS,* 1984, vol. 61 (7), 1261-1265
• **MORR C.V. ; GERMAN, B. ; KINSELLA, J.E. ; REGENSTEIN, J.M. ; VAN BUREN, J.P. ; KILARA, A. ; LEWIS, B.A. ; MANGINO, M.E.** A Collaborative Study to Develop a Standardized Food Protein Solubility Procedure. *Journal of Food Science,* 1985, vol. 50, 1715-1718
• Approved methods of the AACC. American Association of Cereal Chemists, 2000
• **LUDWIG I. ; LUDWIG, E. ; PINGEL B.** Eine Mikromethode zur Bestimmung der Fettbindkapazität. *Nahrung/Food,* 1989, vol. 33 (1), 99
• **WÄSCHE, A. ; MÜLLER, K. ; KNAUF, U.** New processing of lupin protein isolates and functional properties. *Nahrung/Food,* 2001, vol. 45, 393-395
• **V., MÜNSTER.** DGF-Einheitsmethoden. Stuttgart: WVG, 2004